# EUROPEAN PATENT SPECIFICATION

(11) **EP 1 512 683 B1**
(45) Date of publication and mention of the grant of the patent: **31.08.2011**
(21) Application number: 04100891.3
(22) Date of filing: 09.03.1999
(51) Int. Cl.: C07D 211/22, A61K 31/435, C07D 221/22, C07D 211/58, A61K 31/445, C07D 221/08

(54) **Novel opiate compounds, methods of making and methods of use**
Neue Opiate, Herstellungsverfahren und Verwendungen
Nouveaux composés opiacés et leurs procédés de préparation et d'utilisation

(30) Priority: 10.03.1998 US 77402 P; 10.11.1998 US 107902 P
(43) Date of publication of application: 09.03.2005
(62) Divisional of application: 99912345.8
(73) Proprietor: RESEARCH TRIANGLE INSTITUTE, Research Triangle Park, NC 27709 (US)
(72) Inventor: Carroll, Frank, Ivy, Durham, NC 27712 (US); Mascarella, S., Wayne c/o Res. Triangle Institute, Research Triangle Park, NC 27709-2194 (US); Thomas, James, B, Chapel Hill, NC 27516 (US)
(74) Representative: Poulin, Gérard

(56) References cited:
- EP-A- 0 018 077
- US-A- 5 270 328
- US-A- 5 319 087
- THOMAS J B ET AL: "IDENTIFICATION OF AN OPIOID KAPPA RECEPTOR SUBTYPE-SELECTIVE N-SUBSTITUENT FOR (+)-(3R,4R)-DIMETHYL-4-(3-HYDROXYPHENYL) PIPERIDINE" JOURNAL OF MEDICINAL CHEMISTRY, AMERICAN CHEMICAL SOCIETY. WASHINGTON, US, vol. 41, 1998, pages 5188-5197, XP002919013 ISSN: 0022-2623
- THOMAS J B ET AL: "N-SUBSTITUTED OCTAHYDRO-4A-(3-HYDROXYPHENYL)-10A-METHYL- BENZOÄGÜISOQU INOLINES ARE OPIOID RECEPTOR PURE ANTAGONISTS" BIOORGANIC & MEDICINAL CHEMISTRY LETTERS, OXFORD, GB, vol. 8, 1998, pages 3149-3152, XP002919014 ISSN: 0960-894X
- FROIMOWITZ M. ET AL.: "Phenylmorphans and Analogues: Opioid Receptor Subtype Selectivity and Effect of Conformation on Activity" JOURNAL OF MEDICINAL CHEMISTRY, vol. 35, no. 9, 1992, pages 1521-1525, XP002203667
- THOMAS J B ET AL: "A Stereoselective Synthetic Approach to N-Alkyl-4beta-methyl-5-phenyl morphans" TETRAHEDRON LETTERS, ELSEVIER SCIENCE PUBLISHERS, AMSTERDAM, NL, vol. 40, no. 3, 15 January 1999 (1999-01-15), pages 403-406, XP004151342 ISSN: 0040-4039
- THOMAS, J. B. ET AL.: "N-substituted 9.beta.-Methyl-5-(3-hydroxyphenyl)morphans Are Opioid Receptor Pure Antagonists" JOURNAL OF MEDICINAL CHEMISTRY, vol. 41, no. 21, 1998, pages 4143-4149, XP002203671
- AWAYA H ET AL: "RACEMIC AND OPTICALLY ACTIVE 2,9-DIMETHYL-5-(M-HYDROXYPHENYL) MORPHANS AND PHARMACOLOGICAL COMPARISON WITH THE 9-DEMETHYL HOMOLOGUES" JOURNAL OF MEDICINAL CHEMISTRY, AMERICAN CHEMICAL SOCIETY. WASHINGTON, US, vol. 27, no. 4, 1984, pages 536-539, XP002919015 ISSN: 0022-2623
- BERTHA, C. M. ET AL.: "Probes for arcotic Receptor-Mediated Phenomena. 20. Alteration of Opioid Receptor Subtype Selectivity of the 5-(3-Hydroxyphenyl)morphans by Application of the Message-Address Concept: Preparation of .delta.-Opioid Receptor Ligands" JOURNAL OF MEDICINAL CHEMISTRY, vol. 38, no. 9, 1995, pages 1523-1537, XP002203668
- BERTHA, C. M. ET AL.: "A Marked Change of Receptor Affinity of the 2-Methyl-5-(3-hydroxyphenyl)morphans upon Attachment of an (E)-8-Benzylidene Moiety: Synthesis and Evaluation of a New Class of .sigma. Receptor Ligands" JOURNAL OF MEDICINAL CHEMISTRY, vol. 37, no. 19, 1994, pages 3163-3170, XP002203669
- ONG, H. H. ET AL.: "Phenylmorphan Agonists-Antagonists" JOURNAL OF MEDICINAL CHEMISTRY, vol. 17, no. 1, 1974, pages 133-134, XP002203670

## Description

### BACKGROUND OF THE INVENTION

### Field of the Invention

The present invention relates to novel opioid receptor antagonists and agonists.

### Description of the Bacground

The opioid receptor system has been extensively studied over the past eight decades, driven primarily by a search for analgesics that do not possess the abuse potential associated with morphine. While these studies were unsuccessful, our understanding of the opioid system has increased tremendously. A significant breakthrough in our understanding of this system came about as a realization that the pharmacology of opioids is receptor based. From this vantage point, the focus of research turned to identifying receptor subtypes with the ultimate goal of assigning specific physiological function to individual receptors. Today, the receptor system is known to be composed of the three distinct subtypes OP₁, OP_{2,}and OP₃ (delta, kappa and mu), as each of these have been cloned and been shown to derive from three different chromosomes. For a discussion of opioid receptors, see Kirk-Othmer Encyclopedia of Chemical Technology, Volume 17, Fourth Edition, 1996, pp. 858-881. There is however less however as to the number of subtypes within each of the main branches and while much has been learned along these lines, the process of assigning function to subtypes is still an area of active investigation.

The opioid receptor system has been extensively studied over the past eight decades driven primarily by a search for analgesics that do not possess the abuse potential associated with morphine. While this effort has been unsuccessful to date, recent studies have highlighted the delta opioid receptor system as holding the greatest potential for success. Principally, agonists acting through the delta opioid receptor have been shown to modulate pain while minimizing many of the side-effects associated with morphine which acts primarily at the mu opioid receptor. These unwanted side-effects include physical dependence, respiratory depression, and gastrointestinal motility problems. These findings have led to a dramatic increase in the research efforts directed toward the production of potent, highly delta receptor selective agonists. The bulk of this effort has been in discovering small molecules as opposed to peptides due to their enhanced stability *in vivo* and their ability to penetrate the central nervous system.

### I.

The discovery of potent, highly receptor-selective opioid pure antagonists has been a goal of medicinal chemists for many years.^{1,2} As molecular probes, antagonists have served as useful tools in the study of both the structure as well as the physiological functions of the highly complex opioid receptor system. Much has been accomplished as evidenced by the elegant work of Portoghese and coworkers over the past decade which ultimately has led to the discovery of the naltrexone-based kappa and delta receptor subtype-selective antagonists norbinaltorphimine³ (1, nor-BNI) and naltrindole⁴ (2, NTI), respectively. Following Portoghese's lead, workers at SmithKline Beecham recently reported that the octahydroisoquinoline (3, SB 205588) was a second-generation, highly potent and selective delta antagonist formally derived from naltrindole fragmentation.⁵ One specific research aim has been the discovery of opioid receptor selective reversibly binding ligands from the N-substituted (+)-(3R,4R)-dimethyl -4-(3-hydroxyphenyl)piperidine (4a) class of compounds that display pure antagonist activity.⁶ These compounds will be useful as molecular probes for the opioid receptor as well as potential drug candidates for the treatment of substance abuse and other CNS disorders.⁷ While mu antagonists have for years been used in drug abuse therapy, recent findings suggest that kappa antagonists could provide a more effective, long-tasting treatment strategy.⁸ A great variety of N-substituted derivatives of 4a has been prepared, but until the recent demonstration of mu selectivity for 5a, ⁹ none had shown selectivity between the opioid receptor subtypes. Since the pure antagonist activity of these compounds is not dependent on the N-substituent, multiple changes to this part of the molecule would be expected to affect binding affinity and possibly receptor selectivity but not alter its fundamental antagonist character. This feature distinguishes this class of antagonist from the morphone-based compounds, which display pure antagonist behavior only with N-substituents such as allyl or cyclopropylmethyl but not methyl, ethyl, or phenethyl.¹⁰ It is currently believed that the N-substituent in 4a interacts with a lipophilic binding domain which has been described as either very large or quite malleable since a multitude of different types of N-substituent changes provided ligands displaying high binding affinity.¹¹ It has also been determined that maximum potency and selectivity for the mu opioid receptor is achieved when the N-substituent incorporates a lipophilic entity (phenyl or cyclohexyl ring) separated from the piperidine nitrogen by three atoms as illustrated by compounds 5a-d.^{9,11} The synthesis of κ-selective compounds remains an important goal. compounds 5a-d.^{9,11}

### II.

Derivatives of N-substituted (±)-trans-3,4-dimethyl-4-(3-hydroxyphenyl)piperidine, such as 6 and 7, are known to posses nonselective potent opioid pure antagonist activity. ¹²⁻¹⁶ Early investigations of the phenylpiperidine class of opioid antagonists identified the 3-methyl substituent and its trans relative relationship to the 4-substituent as being both necessary and sufficient to impart antagonist activity to the agonist 4-(3-hydroxyphenyl)piperidine. ¹² This feature distinguished the phenylpiperidines from the oxymorphones which rely on particular N-substituents (i.e. allyl, cyclopropylmethyl) for expression of opioid antagonist activity. ¹⁷ Further studies demonstrated that the N-substituent in the phenylpiperidine antagonists controls their potency and efficacy.¹⁵ Accordingly, there remains a need for compounds which have similar therapeutic effects as the trans-3 ,4-dimethyl-4-(3-hydroxyphenyl)piperidines, but are based on different structural element.

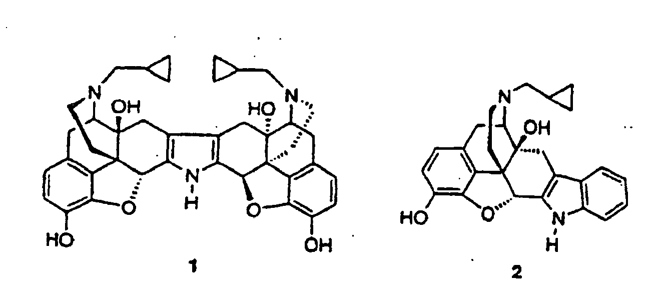

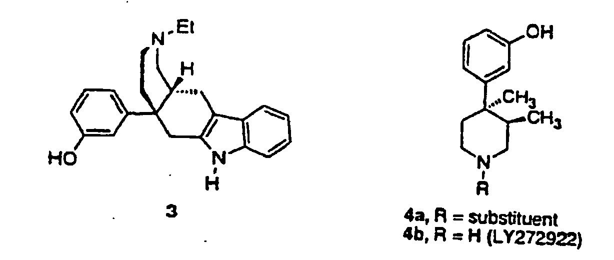

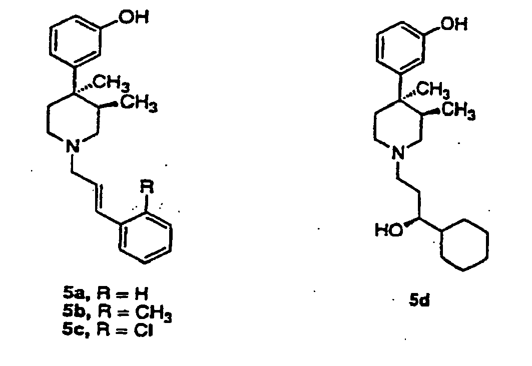

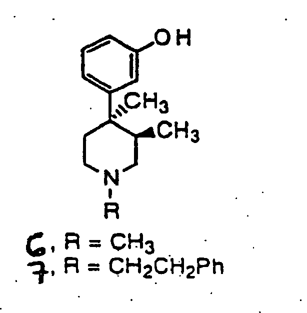

### III.

Numerous structural types of opioid agonists have been discovered, and several such as methadone, meperidine, fentanyl, and pentazocine as well as others have become important drugs for the treatment of pain.¹⁰ However, there are only a few structural types that show potent, opioid pure antagonist activity.^{10,7} A resurgence in heroin use in recent years coupled with the demonstrated effectiveness of opioid antagonists for the treatment of other substances of abuse has spurred new interest in the development of novel antagonists for opioid receptors.¹⁶

The oxymorphone-related compounds such as naloxone (**8a**) and naltrexone (**8b**), where the antagonist activity is dependent upon the N-substituent, have received considerable attention over the past few decades.¹⁰ For example, pioneering studies by Portoghese and coworkers lead to the development of the prototypical kappa and delta opioid receptor antagonists, norbinaltorphimine (**1**, nor-BNI) and naltrindole (2, NTI). In contrast, the N-substituted trans-3,4-dimethyl-(3-hydroxyphenyl)piperidine (**9a-d**) class of pure antagonist has received relatively little attention. Studies with the N-methyl analog 9a as well as many other N-substituted analogs such as **9b, 9c** (LY255582), and **9d** showed that the pure antagonist activity was dependent on the 3-methyl substituent and its trans relative relationship to the 4-methyl substituent on the piperidine ring and, unlike the oxymorphone class, was independent of the nature of the N-substituent.^{7,16,17,6,13,14}. Interestingly, the 3,4-dimethyl cis isomer **9e** was found to be a mixed agonist-antagonist. May and coworkers¹⁸ reported that 2,9α-dimethyl-5-(3-hydroxyphenyl)morphan (**10a**), which has the 9-methyl group in a configuration comparable to the cis-3,4-dimethyl-4-(3-hydroxyphenyl)piperidine (**9e**) with the 5-(3-hydroxyphenyl) group locked in an equatorial conformation relative to the piperidine ring in the morphan structure, was a weak but pure antagonist.

Neither 2,9β-dimethyl-5-(3-hydroxyphenyl)morphan (**10b**) nor 2,4β-dimethyl-5-(3-hydroxyphenyl)morphan (**10g**) have not been reported, due to a lack of synthetic accessibility to these structural isomers. Accordingly, the successful synthetic preparation of 2,9β-morphans and 2,4β-morphans remains an important goal of in the field opioid receptor-binding compounds.

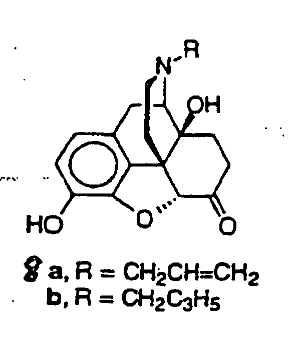

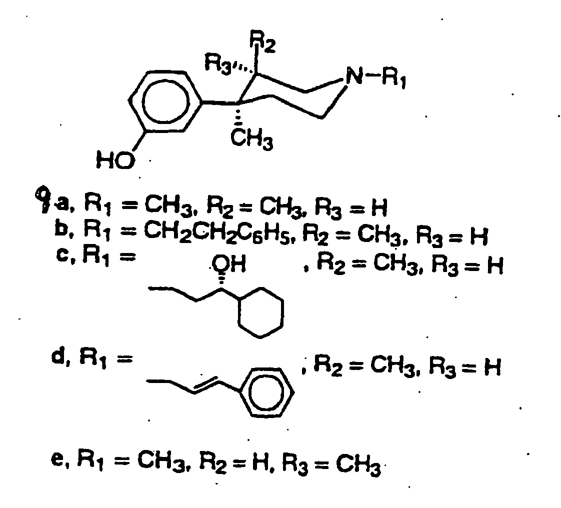

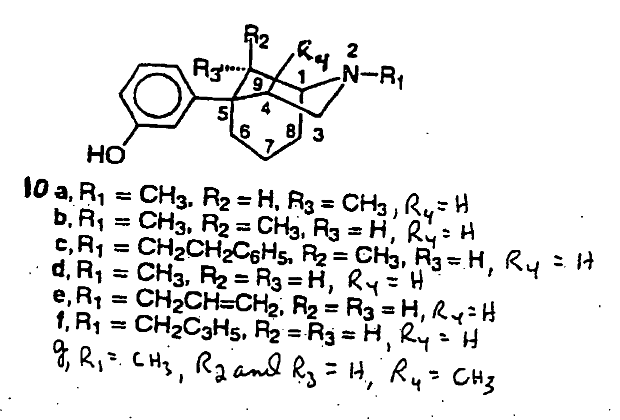

### IV.

In search of analgesics possessing a reduced side-effect profile relative to morphine, much effort has been expended towards finding opioids which operate via δ or κ opioid receptors as opposed to the µ opioid receptor which meditates the actions of morphine and its congeners.¹⁰ *BW373U86 (11)¹⁹ and SNC-80 (**12**)²⁰ represent one class of opioid agonists discovered to be selective for the δ opioid receptor. Due to the lack of a clear opioid message substructure (i.e., a tyramine component similar to the enkephalins), compounds **11** and **12** have been referred to as non-classical opioid ligands.⁵ The piperazine subunit of **11** and **12** is not commonly found in compounds showing activity at the opioid receptors. In contrast, piperidine ring compounds are found in many different classes of opioids.²⁷ If the internal nitrogen atom in compounds **11** or **12** is transposed with the benzylic carbon, piperidine ring analogs such as **13** would be obtained. Even though there are common structural elements between structures **11** or **12** and **13,** the expected difference between in basicity between the piperidinyl amino group of **11** or **12** and the di-phenyl substituted amine of **13** is sufficient such that it cannot be predicted whether similarity to suggest that **13** would interact with opioid receptors similar to **11** or **12.** It is also interesting to note that compound **13** has some structural elements in common with cis-3-methylfentanyl (**14**),^{21,22} a non-classical opioid ligand selective for the mu opioid receptor. Accordingly, the preparation of compound **13** and related structures remains an important goal.

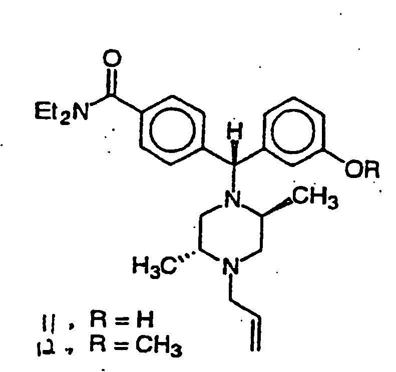

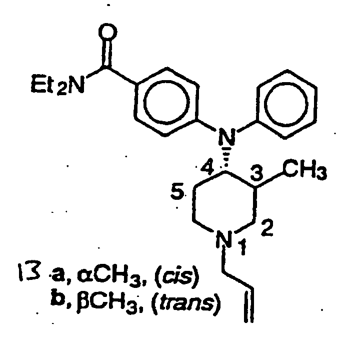

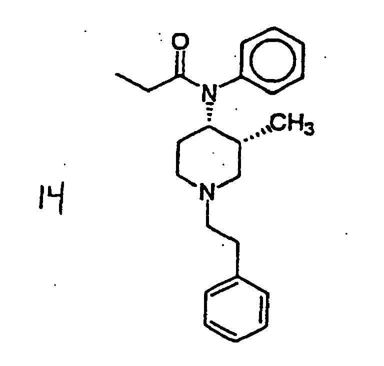

### References

(1) Dhawan, B.N.; Cesselin, F.; Raghubir, R.; Reisine, T.; Bradley, P.B.; Portoghese, P.S.; Hamon, M. International Union of Pharmacology. XII. Classification of opioid receptors. Pharmacol. Rev. 1996, 48, 567-592.
(2) Martin, W.R The evolution of concepts of opioid receptors. In The Opiate Receptors, Pasternak, G.W. Eds.; Humana-Press Inc.: New Jersey, 1988, pp. 3-22.
(3) Portoghese, P:S.; Nagase, H.; Lipkowski, A.W.; Larson, D.L.; Takemori, A.E. Binaltorphimine-related bivalent ligands and their kappa opioid receptor antagonist selectivity [published erratum appears in J. Med. Chem. 1988 Oct;31(10):2056]. J. Med. Chem. 1988, 31, 83 6-841.
(4) Portoghese, P.S. An approach to the design of receptor-type-selective non-peptide antagonists of peptidergic receptors: δ opioid antagonists. J. Med. Chem. 1991, 34(6), 1757-1762.
(5) Dondio, G.; Ronzoni, S.; Eggleston, D.S.; Artico, M.; Petrillo, P.; Petrone, G.; Visentin, L.; Farina, C.; Vecchietti, V.; Clarke, G.D. Discovery of a novel class of substituted pyrrolooctahydroisoquinolines as potent and selective δ opioid agonists, based on an extension of the message-address concept. J. Med. Chem. 1997, 40, 3192-3198.
(6) Zimmerman, D.M.; Nickander, R.; Homg, J.S.; Wong, D.T. New structural concepts for narcotic antagonists defined in a 4-phenylpiperidine series. Nature 1978, 275, 332-334.
(7) Zimmerman, D.M.; Leander, J.D. Invited perspective, selective opioid receptor agonists and antagonists: Research tools and potential therapeutic agents. J. Med. Chem. 1990, 33, 895-902.
(8) Rothman, RB.; Gorelick, D.A.; Eichmiller, P.R.; Hill, B.H.; Norbeck, J.; Liberto, J.G. An open-label study of a functional opioid kappa antagonist in the treatment of opioid dependence. In Problems of Drug Dependence, 1997: Proceedings of the 59th Annual Scientific Meeting, The College on Problems of Drug Dependence; Inc., Harris, L.S. Eds.; U. S. Department of Health and Human Services: Rockville, MD, 1997; Vol. 178, pp. 309.
(9) Thomas, J.B.; Mascarella, S.W.; Rothman, R.B.; Partilla, J.S.; Xu, H.; McCullough, K.B.; Dersch, C.M.; Cantrell, B.E.; Zimmerman, D.M.; Carroll, F.I. Investigation of the N-substituent conformation governing potency and µ receptor subtype-selectivity in (+)-(3R,4R)-dimethyl-4-(3-hydroxyphenyl)piperidine opioid antagonists. J. Med. Chem. 1998, 41(11), 1980-1990.
(10) Aldrich, J.V. Analgesics. In Burger's Medicinal Chemistry and Drug Discovery, Wolff, M.E. Eds.; John Wiley & Sons, Inc.: 1996; Vol. 3: Therapeutic Agents.
(11) Mitch, C.H.; Leander, J.D.; Mendelsohn, L.G.; Shaw, W.N.; Wong, D.T.; Cantrell, B.E.; Johnson, B.G.; Reel, J.K.; Snoddy, J.D.; Takemori, A.E.; Zimmerman, D.M. 3,4-Dimethyl-4-(3-hydroxyphenyl)piperidines: Opioid antagonists with potent anorectant activity. J. Med. Chem. 1993, 36(20), 2842-2850.
(12) Zimmerman, D.M.; Smits, S.; Nickander, R. Further investigation of novel 3-methyl-4-phenylpiperidine narcotic antagonists. In Proceedings of the 40th Annual Scientific Meeting of the Committee on Problems of Drug Dependence, 1978, pp. 237-247.
(13) Zimmerman, D.M.; Smits, S.E.; Hynes, M.D.; Cantrell, B.E.; Leander, J.D.; Mendelsohn, L.G.; Nickander, R. Drug Alcohol Depend. 1985, 14, 381-402.
(14) Mitch, C.H.; Leander, J.D.; Mendelsohn, L.G.; Shaw, W.N.; Wong, D.T.; Zimmerman, D.M.; Gidda, S.J.; Cantrell, B.E.; Scoepp, D.D.; Johnson, B.G.; Leander, J.D. J. Med. Chem. 1994, 37, 2262-2265.
(15) Evans, D.A.; Mitch, C.H.; Thomas, R.C.; Zimmerman, D.M.; Robey, R.L. Application of metalated enamines to alkaloid synthesis. An expedient approach to the synthesis of morphine-based analgesics. J. Am. Chem. Soc. 1980, 102, 5955-5956.
(16) Kreek, M.J. Opiates, opioids and addiction. Mol. Psychiatry 1996, 1(3), 232-254.
(17) Zimmerman, D.M.; Gidda, J.S.; Cantrell, B.E.; Schoepp, D.D.; Johnson, B.G.; Leander, J.D. Discovery of a potent, peripherally selective trans-3,4-dimethyl-4-(3-hydroxyphenyl)piperidine opioid antagonist for the treatment of gastrointestinal motility disorders. J. Med Chem. 1994, 37(15), 2262-2265.
(18) Awaya, H.; May, E.L.; Aceto, M.D.; Merz, H.; Rogers, M.E.; Harris, L.S. Racemic and optically active 2,9-dimethyl-5-(m-hydroxyphenyl)morphans and pharmacological comparison with the 9-demethyl homologues. J. Med Chem. 1984, 27, 536-539.
(19) Chang, K.J.; Rigdon, G.C.; Howard, J.L.; McNutt, R.W. A novel potent and selective nonpeptidic delta opioid receptor agonist, BW373U86. J. Pharm. Exp. Ther. 1993, 267, 852-857.
(20) Calderon, S.N.; Rothman, R.B.; Porreca, F.; Flippen-Anderson, J.L.; McNutt, R.W.; Xu, H.; Smith, L.E.; Bilsky, E.J.; Davis, P.; Rice, K.C. Probes for narcotic receptor mediated phenomena. 19. Synthesis of (+)-4-[(αR)-α-(2S,SR)-4-allyl-2,5-dimethyl-1-piperazinyl)-3-methoxybenzyl]-N,N-diethylbenzamide (SNC 80): A highly selective, nonpeptide δ opioid receptor agonist. J. Med. Chem. 1994, 37,2125-2128.
(21) Van Bever, W.F.; Niemegeers; C.J.E.; Janssen, P.A.J. Synthetic analgesics. Synthesis and pharmacology of the diasteoisomers of N-(3-methyl-1-(2-phenylethyl)-4-piperidyl)-N-phenylpropanamide and N-(3-methyl-1-(1-methyl-2-phenylethyl)-4-piperidyl)-N-phenylpropanamide. J. Med. Chem. 1974,17(10), 1047-1051.
(22) Xu, H.; Kim, C.-H.; Zhu, Y.C.; Weber, R.J.; Rice, K.C.; Rothman, R.B. (+)-cis-Methylfentanyl and its analogs bind pseudoirreversibly to the mu opioid binding site: Evidence for pseudoallosteric modulation. Neuropharmacology 1991, 30, 455-462.

### SUMMARY OF THE INVENTION

It is an object of the present invention to provide novel compounds which bind to opioid receptors.

It is another object of the present invention to provide novel compounds which are opioid receptors antagonists that bind with high affinity.

It is another object of the present invention to provide novel opiates that are selective for the Kappa receptor as compared to the delta and mu receptors.

It is another object of the present invention to provide novel opiates that are selective for the mu and kappa receptors as compared to the delta receptor.

It is another object of the present invention to provide novel opiates that are selective for the delta receptor as compared to the mu and kappa receptors.

It is another object of the present invention to provide novel opiates that are pure antagonists at the mu, delta and kappa receptors.

The objects of the present invention may be accomplished with compounds represented by formula (I), or pharmaceutically acceptable salts thereof: 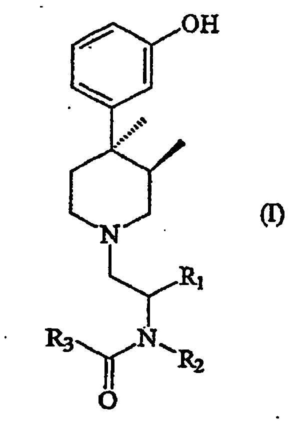 wherein
R₁ is hydrogen, a methyl group, an isopropyl group, isobutyl group, a cyclohexyl group, a secondary butyl group, a benzyl group, or a phenyl group ;
R₂ is hydrogen, or a methyl group; and
R₃ is 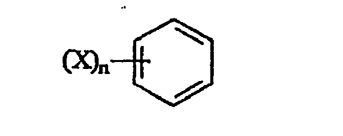 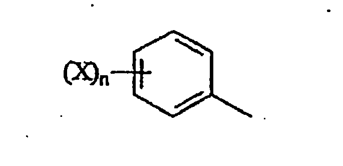 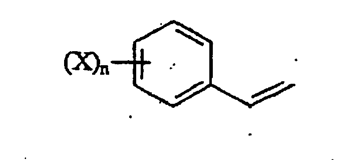 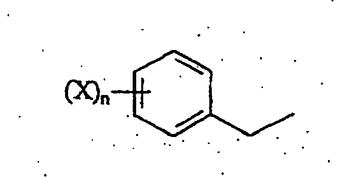 each X is, independently -OH, OMethyl, OEthyl, OBenzyl, Ombutyl, OPhenyl, Methyl, Isopropyl, Butyl, phenyl, N(CH₃)₂, C(O) NH₂, F, Cl;
1 or 2. n ; and

The objects of the present invention may especially be accomplished by the compounds disclosed in claims 2, 3, 4, 5 and 7.

The objects of the present invention may also be accomplished by the compounds disclosed in claim 6.

### (II)

A more complete appreciation of the invention and many of the attendant advantages thereof will be readily obtained as the same becomes better understood by reference to the following detailed description when considered in connection with the accompanying drawings.

### BRIEF DESCRIPTION OF THE DRAWINGS

Figure 3: Synthesis of compounds (7) as described in Example 1.

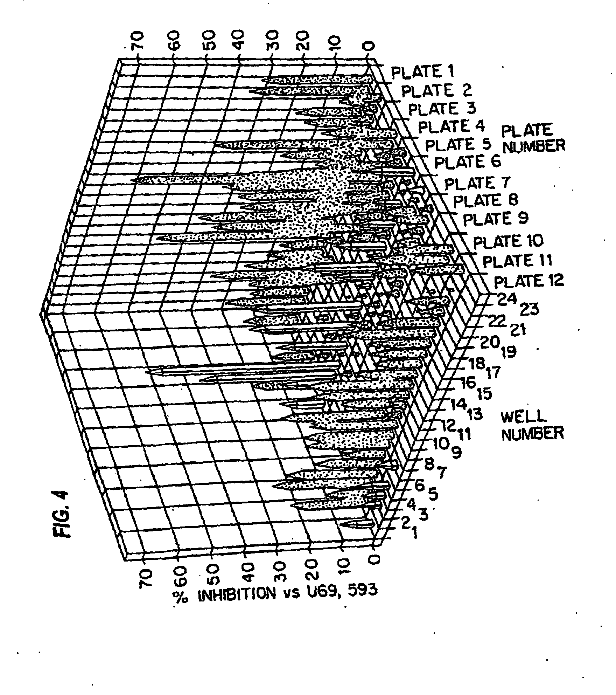

Figure 4: Data from screening of library described in Example 1 at 100 nM against the kappa-selective ligand [³H]U69,593 (percent inhibition).
Figure 5 : Comparison of ratios of radioligand binding and GTPγS assays for compound 8, naltrexone, nor-BNI, 5d, and 5a-c described in Example 1, the N-trans-cinnamyl derivatives of 4b. The radioligand and GITγS binding data for 5a-d were taken from ref. 9 cited in Example 1.
Figure. 7: Structural representation of (a) Naltrexone, (b) 3,4-dimethyl-4-(3-hydroxyphenyl)piperidine, and (c) 8a-methyl-4a-(3-hydroxyphenyl)-octahydrobenzo[e]isoquinolino (Example 2).

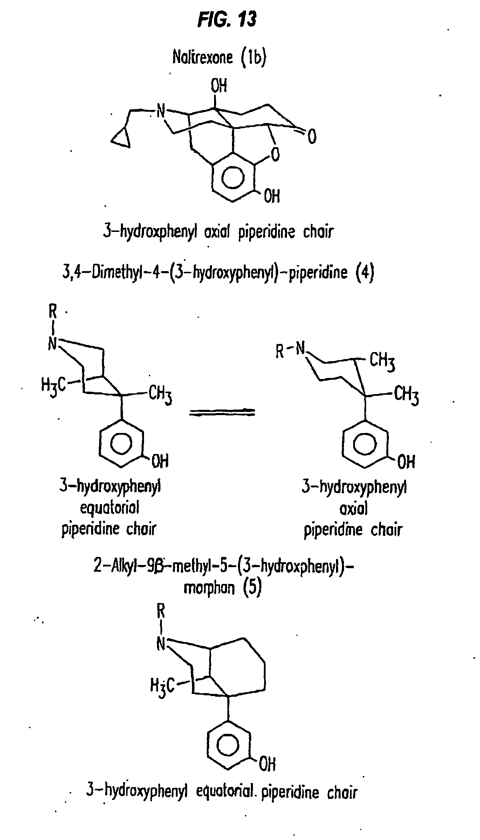

Figure 13: Conformational representation of naltrexone (1b), N-substituted 3,4-dimethyl-4-(3-hydropheny)piperidine, and 2-alkyl-9β-5-(3-hydroxyphenyl)morphan. These compounds are described in Example 4.

### DETAILED DESCRIPTION OF THE INVENTION

The present invention relates to a group compounds that contain a piperidinyl group. The inventive compounds have been found to have a variety of different activities when bound to opioid receptors.

### Compounds pf Formula (I)

In formula (I), R₁ is as already defined above.
R₂ in formula (I) is as defined above.
Preferably, R₂ is hydrogen R₃ in formula (I) is one of the following groups:

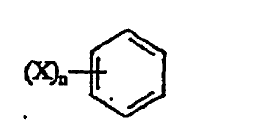

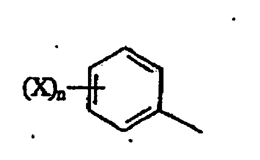

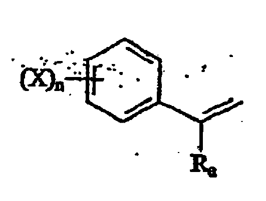

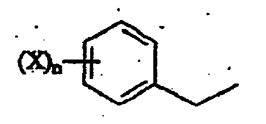

In these groups, the phenyl ring is substitued with 1, or 2, X groups, each X is, independent as already defined above,

Preferred X groups are chlorine, fluorine,-OH, and -OCH₃. Preferably, a is 1. The X group(s) may be located at the *ortho, meta* and *para* positions. The *para* position is preferred, especially when X is -OH.

The absolute configuration of the carbon atom to which R, is bonded may be (R) or (S). The (S) configuration is preferred.

The compounds of formula (I) are preferably opiates with preferential affinity for the µ/κ opioid receptors and comparably less affinity for δ receptors. In a preferred embodiment, these compounds are pure antagonists. The ratio of affinity for the δ receptor to the κ receptor (δ/κ) may be at least 1.5, preferably at least 2.0, more preferably at least 20, still more preferably at least 100, even still more preferably at least 750 and most preferably at least 800. The µ/κ ratio may be 0.002 to 500.

The compounds of formula (I) may be prepared using well-known synthetic techniques by condensing an acid of the formula R₃-CO₂H with an amine represented by the formula:

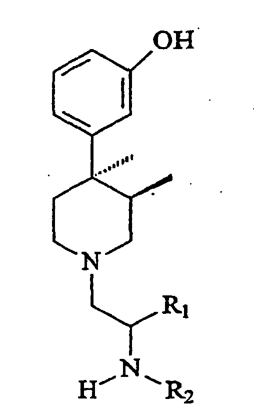

The acid is preferably converted into an activated ester in order to couple with the amine. A BOP ester is preferred. In a particularly preferred embodiment, a variety of compounds within the scope of formula (I) may be simultaneously synthesized and evaluated using well-established combinatorial synthesis techniques, for example, as described in Example 1.

Compounds (I) and compounds of claim 6 may be in the form of a pharmaceutically acceptable salt via protonation of the amine with a suitable acid. The acid may be an inorganic acid or an organic acid. Suitable acids include, for example, hydrochloric, hydroiodic, hydrobromic, sulfuric, phosphoric, citric, acetic and formic acids.

The receptor selectivities discussed above are determined based on the binding affinities at the receptors indicated.

The compounds of the present invention may be used to bind opioid receptors. Such binding may be accomplished by contacting the receptor with an effective amount of the inventive compound. Of course, such contacting is preferably conducted in a aqueous medium, preferably at physiologically relevant ionic strength, pH, etc.

The inventive compounds may also be used to treat patients having disease states which are ameliorated by binding opioid receptors. Such diseases states include heroin addition, pain, i.e., the compounds are used as analgesics. The compounds of the inventive may also be used to reverse mu-induced respiratory depression, as cytostatica agents, as antimigraine agents, as immunomodulators, as immunosuppressives, as antiarthritic agents, as antiallergic agents, as virucides, to treat diarrhea, as antidepressants, as uropathic agents, as antitussives, as antiadditive agents, as anti-smoking agents, to treat alcoholism, as hypotensive agents, or to treat obesity.

The compounds may be administered in an effective amount by any of the conventional techniques well-established in the medical field. For example, the compounds may be administered orally, intraveneously, or intramuscularly. When so administered, the inventive compounds may be combined with any of the well-known pharmaceutical carriers and additives that are customarily used in such pharmaceutical compositions. For a discussion of dosing forms, carriers, additives, pharmacodynamics, etc., see Kirk-Othmer Encyclopedia of Chemical Technology, Fourth Edition, Vol. 18, 1996, pp. 480-590, incorporated herein by reference. The patient is preferably a mammal, with human patients especially preferred.

Having generally described this invention, a further understanding can be obtained by reference to certain specific examples which are provided herein for purposes of illustration only and are not intended to be limiting unless otherwise specified. In each of the Examples, the numbering of compounds and references cited are specific to each Example.

### EXAMPLES

### Example 1

### Identification of Opiates selective for the Opioid Receptors

### Summary

A three-component library of confounds was prepared in parallel using multiple simultaneous solution phase synthetic methodology. The compounds incorporated a (+)-(3R,4R)-dimethyl-4-(3-hydroxphenyl)piperidine group as one of the monomers. The other two monomers, which included N-substittuted or unsubstitued Boc protected amino acids and a range of substituted aryl carboxylic acids, were selected to add chemical diversity. Screening of these compounds in competitive binding experiments with the kappa opioid receptor selective ligand [³H]U69,593 led to the identification of a κ opioid receptor selective ligand, N-{(2'S)-[3-(4-hydroxyphenyl)propanamido]-3'-methylbutyl}-(3R,4R)-dimethyl-4(3-hydroxyphenyl)piperidine (8, RTI-5989-29). Additional SAR studies suggested that 8 possesses lipophilic and hydrogen bonding sites that are important to its opioid receptor potency and selectivity. The sites appear to exist predominantly within the kappa receptor since the selectivity arises from a 530-fold loss of affinity of 8 for the mu receptor and an 18-ford increase in affinity for the kappa receptor relative to the mu selective ligand, (+)-N--[trans-4-phenyl-2-butenyl]-(3R,4R)-dimethyk-4-(3-hydroxyphenyl)piperidine (5a). THis degree of selectivity observed in the radioligand binding experiments was not observed in the functional assay. According to its ability to inhibit agonist stimulated binding of ^{[35}S]GTPγS at all three opioid receptors, compound 8 behaves as a mu/kappa opioid receptor pure antagonist with negligible affinity for the delta receptor.

### Chemistry

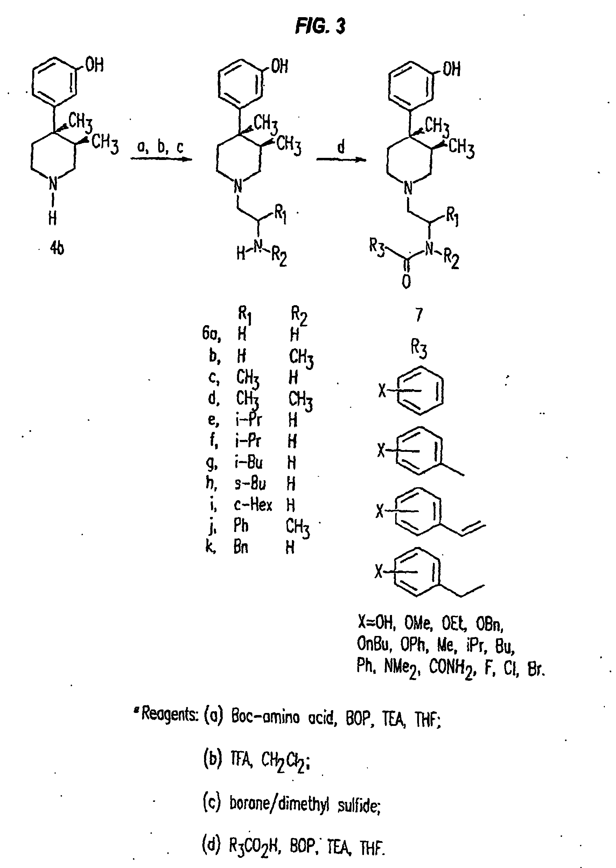

Coupling of (+)-(3R,4R)-dimethyl-4-(3-hydroxyphenyl)piperidine (4b) (Figure 3) with an appropriate tert-butoxycarbonyl-protected amino acid (Boc-protected) followed by removal of the Boc-protecting group with trifuoroacetic acid (IFA) in methylene chloride followed by reduction using a tetrahydrofuran (THF) solution of borane-dimethyl sulfide complex gave the intermediate amines (6a-k) in 15-78% yields (Figure 3): These intermediates **6** were subjected to column chromatography or crystallization as necessary to obtain pure compounds. The final products (7) were prepared in scintillation vials via amide bond formation by coupling with a wide variety of commercially available carboxylic acids. A representative list of such carboxylic acids follows the Experimental section of this Example. Benzotriazol-1-yl-oxy-tris-(dimethylamino)phosphonium hexafluorophosphate (BOP reagent) in THF was employed as the coupling reagent which provided very clean products after aqueous work-up. These compounds were used directly in screening without additional purification. Pure compounds for further SAR analysis were obtained by purification of library samples or by single compound synthesis by conventional synthetic methodology and characterized by MS, ¹H NMR, and elemental analyses.

### Results and Discussion

The results from the screening of the 288-compound library in competitive binding against the kappa opioid receptor selective ligand [³H]U69,593 are illustrated graphically in Figure 6. Several compounds showed significant inhibition of radioligand binding at 100 nM with **8** (plate 4, well 20, 71%) being the best (Figure 6). The data for % inhibition of [³H]U69,593 binding by selected library compounds **8-23** at 100 nM are listed in Table 1.

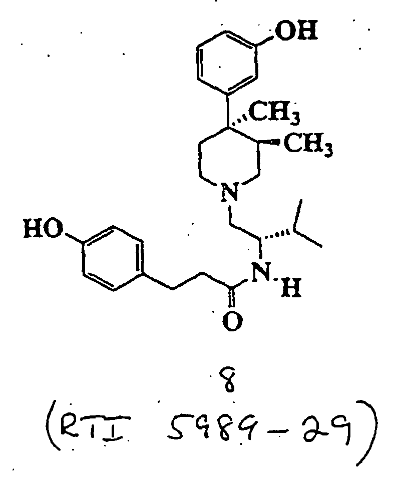

A comparative analysis of the structures related to compounds **9-23**, which have less binding affinity relative to **8**, readily illustrates the importance for kappa receptor binding of each structural subunit of group R₃ (Table 1). Compound **9**, a diastereomer of **8**, where the carbon to which the R₁ isopropyl group is connected has the opposite stereochemistry, shows less binding affinity (11%) for the opioid kappa receptor. The sensitivity to orientation (R or S) at this stereogenic center suggests that the isopropyl group may be working in tandem with another structural feature of the R₃ unit to both increase binding in **8** and decrease binding in **9**. The difference in affinity of compounds **8** (71%) and **10** (28%) suggests that the 4-hydroxyl substituent in **8** is more effective for high kappa binding affinity. Furthermore, the weaker inhibition displayed by compounds **11** (20%) and 12 (25%) possessing meta and ortho hydroxyl substituents respectively, pinpoints the para placement of the para-hydroxyl group as the optimum position. The fact that compound **19**, which lacks the isopropyl group but has the para-hydroxyphenylpropionic substituent, shows less affinity (11% vs. 71 %) relative to **8**, adds additional support to the importance of the R₁ isopropyl and 4-hydroxyphenyl groups to the kappa-selective binding. The low affinity of compound **20** (20%) which has a methyl substituent in position (R₁) shows that a methyl group may be less effective than the isopropyl group. This strengthens the notion that both the isopropyl group (R₁) and the 4-hydroxyphenyl group for R₃ are working together to elicit high affinity binding at the kappa opioid receptor in compound **8**. The results for compound **13** (6%) suggests that two methylene groups are more effective between the phenyl ring and the amide carboxyl in diversity element R₃, presumably because the para-hydroxyl group cannot reach its site of interaction in the truncated derivative. Furthermore, the lower inhibition of binding for compound **14** (15%) which incorporates a *trans* double bond in the connecting chain shows that the length of the chain is not optimal to impart high binding affinity, implying that flexibility is also preferred in this carbon chain to provide proper ligand and receptor alignment. The lower affinity of the 4-fluoro derivative **15** (26%) and the 4-methoxy derivative **18** (16%) supports the suggestion that a hydrogen bond exists between ligand **8** and the receptor with compound **8** donating the hydrogen. This is further supported by the lower affinity of the 3,4-dihydroxyl derivative **16** (31 %) which can hydrogen bond internally and the 3-methoxy-4-hydroxy derivative **17** (42%) whose hydrogen bond could be sterically encumbered by interference from an adjacent methoxy group. Interestingly, all compounds having methyl and not hydrogen as the second diversity element R₂, **21** (0%), **22** (1%), and **23** (7%) displayed very low binding affinity usually at baseline (DMSO blank) levels. Apparently, position R₂ is preferably unsubstituted. These results suggest that the amide group may be part of a separate hydrogen-bonding interaction to place the key R₁ isopropyl and R₃ p-hydroxyphenyl rings in their correct positions for strong interaction with the receptor. Alternatively, the N-methyl substituent may be decrease ligand affinity through repulsive steric interactions.

Taken together, the data suggests that the high binding affinity displayed by 8 results from a combination of several structural features present in its N-substituent. These include a 4-hydroxyl group in the pendant phenyl ring of group R₃, the length and flexibility of the carbon chain connecting this ring to the amide carbonyl and the presence of a beta (position R₁) isopropyl group with an S configuration at the adjacent stereogenic center. The data analysis suggests that the principle stabilizing interactions could be related to binding of the hydroxyl and isopropyl substituents with the other atoms of the N-substituent substructure acting to hold these two binding elements in optimum overlapping positions within the receptor site. Alternatively, the isopropyl group could be acting to bias the conformation of molecule to provide the best alignment of the 4-hydroxyphenylpropionic acid side-chain with its binding site.

In order to gain additional SAR information, a pure sample of **8** along with compounds **24-27** which vary at the R₁ position alone was prepared for study. Table 2 lists the Kᵢ values for these derivatives at the mu and kappa opioid receptors along with the Kᵢ values for the mu-selective reference compound **5a**, naltrexone, and the kappa-selective antagonist nor-BNI. The delta receptor assay was not performed for compounds **24-27** as all previous derivatives of **8** had shown no affinity for this receptor subtype. This study revealed that **8** not only actively binds the kappa receptor (Kᵢ = 6.9 nM) but also possessed a 57-fold selectivity for the kappa vs. the mu receptor (Kᵢ = 393 nM) and >870-fold selectivity for the kappa vs. the delta receptor (Kᵢ >5700 nM). Compound **8** thus displays a high degree of opioid kappa receptor subtype selectivity.^{1,2} Nor-BNI (**1**) has a higher affinity for the kappa receptor than **8** and has a greater kappa selectivity relative to the mu receptor. However, **8** is more selective for the kappa receptor relative to the delta receptor. A part of these differences could be due to the use of different tissues and radioligands.

The data for the beta isobutyl substituent compound **24**, which results formally from insertion of a methylene between the isopropyl group and its adjacent stereogenic center of compound **8**, displays a loss of affinity for the kappa receptor while maintaining the same affinity for the mu receptor as compound **8**. The net effect is a loss of selectivity between the mu and kappa receptor subtypes. Compound **26** (R₁=cyclohexyl) shows a similar loss of affinity for the kappa receptor with a gain in affinity for the mu receptor resulting in a similar loss of selectivity. Compound 25 with an R₁ sec-butyl group shows a slight decrease in both kappa and mu potency but retains selectivity, though its magnitude is lower relative to **8**. Compound **27** (R₁=benzyl) displayed a binding profile completely different from that seen in **8** with a tremendous increase in mu potency and concomitant loss of kappa potency. This was not unexpected since compound **27**, prepared from the amino acid phenylalanine, possesses an N-substituent with a phenyl ring separated from the piperidine ring by three methylene groups which are known to favor mu binding.^{1,2} It was for this reason that phenylalanine was excluded from use in the library synthesis. Overall, the behaviors of the various R₁ derivatives of **8** indicate that the size of the lipophilic group in position R₁ is important to both the potency and receptor subtype selectivity of the ligand. Furthermore, the data supports the hypothesis that the isopropyl group in **8** is not simply biasing the conformation of side-chain but is instead interacting with the receptor directly in a ligand stabilizing interaction.

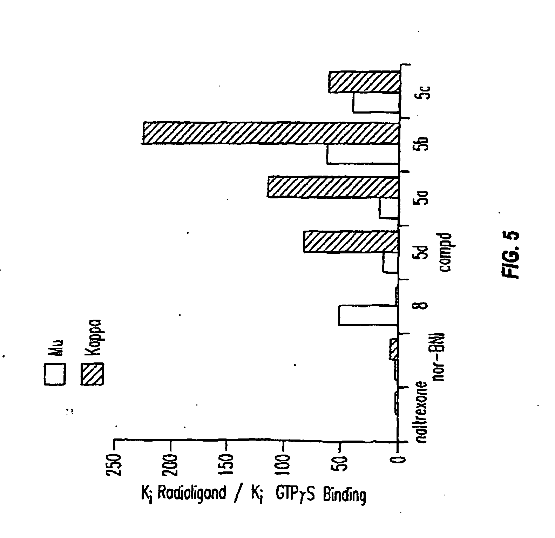

The agonist/antagonist activity of compound **8** was measured by determining its ability to either stimulate or reverse opioid agonist stimulated binding of the nonhydrolyzable GTP analog, [³⁵S]GTPγS, in all three opioid receptor assays (Table 3). Table 3 includes data obtained for naltrexone, the standard nonselective opioid pure antagonist, nor-BNI, the prototypical kappa-selective antagonist, and the potent, mu-favoririg opioid antagonist (**5a**). The kappa selectivity displayed by compound **8** in the inhibition of radioligand binding assay was not observed in the [³⁵S]GTPγS functional assay. This is not an atypical situation; radioligand binding results often differ substantially from those seen in functional assays but this typically involves agonists. The antagonists, naltrexone, normally display Kᵢ (radioligand)/Kᵢ (GTPγS) binding ratios near unity whereas ratios greater than unity have been observed for antagonists of the N-substituted *trans*-3,4-dimethyl-4-(3-hydroxyphenyl)piperidine series.¹ This phenomenon is illustrated graphically in Figure 5. The *trans*-cinnamyl derivatives 5a-c and compound 5d display k₁ (radioligand) K₁(GTRγS) binding ratios greater than unity in the mu and kappa receptor assays which is distinctly different from the response demonstrated by nalttrexone. In the present case compound 8 is found to behave like naltrexone in the kappa receptor assays with a ratio near unity which is far different from the behavior seen for 5a-c and 5d, which show ratios of 118, 228, 63, and 85, respectively. In the mu receptor assay on the other hand, compound 8 with a ratio of 54 behaves like 5a-c and 5d which give ratios of 19,66,43, and 15. This differential response of 8 in the [³⁵S]GTPγS assay is sufficiently large so as to erode the kappa receptor selectivity observed for 8 in the radioligand binding assays. Note that the Kᵢ (radioligand)/kᵢ (GTP) binding ratios for nor-BNI at the mn and kappa receptor are 2.8 and 7.36, respectively.

### Conclusions

The identification of compound 8, which displays a highly selective kappa vs. mu receptor inhibition of radioligand binding profile and a potent mu/kappa opioid antagonist profile, demonstrates the effectiveness of the biased library approach to lead compound generation. Since both the mu and kappa receptors may be important in heroin abuse, compound 8 should be useful as a treatment medication for heroin abuse.

### Experimental section

Melting points were determined on a Thomas-Hoover capillary tube apparatus and are not connected. Elemental analyses were obtained by Atlantic Microlabs, Inc. and are within ±0.4% of the calculated values. All optical rotations were determined at the sodium D line using a Rudolph Research Autopol III polarimeter (1-dm cell). ¹H NMR spectra were determined on a Bruker WM-250 spectrometer using tetramethylsilane as an internal standard. Silica gel 60 (230-400 mesh) was used for all column chromatography. Mass spectral data was obtained using a Finnegan LCQ electrospray mass spectrometer in positive ion mode at atmospheric pressure. All reactions were followed by thin-layer chromatography using Whatman silica gel 60 TLC plates and were visualized by UV, charring using 5% phosphomolybdic acid in ethanol and/or exposure to iodine vapor. All solvents were reagent grade. Tetrahydrofuran and diethyl ether were dried over sodium benzophenone ketyl and distilled prior to use. Methylene chloride was distilled from calcium hydride prior to use.

**General Method for the Introduction of Diversity Elements R₁ and R₂** into **Structure 6**. (+)-(3R,4R)-Dimethyl-4-(3-hydroxyphenyl)piperidine (**4b**) (11.5 mmol), the appropriate Boc-protected amino acid (11.5 mmol) and BOP reagent (11.5 mmol) were combined in THF (150 mL) at room temperature, and to this was immediately added triethylamine (TEA) or diisopropylethylamine (25.3 mmol). After stirring for 1 h, the reaction mixture was poured into ethyl ether (500 mL) and water (150 mL) in a separatory funnel. The mixture was shaken and the aqueous layer removed. This procedure was repeated using 150 mL saturated NaHCO₃ and 150 mL brine. The organic layer was diluted with hexane until 1 cloudy and dried (Na₂SO₄), concentrated under reduced pressure, then dissolved in 100 mL chloroform (stored over K₂CO₃), and concentrated again. This was placed on a high vacuum system to remove residual solvent yielding a foamy yellow/white solid.

After remaining under vacuum on the pump overnight, this unpurified material was dissolved in methylene chloride 45 mL and cooled to -20°C (methanol/ice). To this was added neat trifluoroacetic acid in 10-mL portions over 2 min to give a total addition of 30 mL. The entire mixture was stirred for exactly 30 min and then the cooling bath was removed for exactly 30 min. At this point, the reaction mixture was poured into a 1 L beaker containing a large stir bar and a rapidly agitated mixture of saturated bicarbonate solution (400 mL) and chloroform (150 mL). After completed addition, the pH of the mixture was verified to be 10 and adjusted with solid sodium bicarbonate if necessary. This mixture was poured into a separatory funnel. Any precipitated organic compounds were rinsed into the separatory funnel using a small amount of methanol. The beaker was then rinsed with a small amount of water which was added to the separatory funnel. The layers were agitated, separated, and the aqueous layer extracted five additional times using 3:1 methylene chloride:THF. It was observed that compounds with small groups R₁ required additional extractions and/or sodium chloride saturation of the aqueous layer. The combined organic layers were dried over sodium sulfate and the solvent removed at reduced pressure. The material was then placed on a high vacuum pump to yield a yellow foamy solid.

Unpurified material from the deprotection step was dissolved in THF (150 mL) and cooled to -20°C (methanol/ice). To this stirred mixture was added a solution of borane dimethylsulfide complex, 2M in THF (110 mmol) dropwise. The solution was then heated to reflux and held for 3 h after which time, the solution was cooled to -20°C, and to this was carefully added methanol (72 mL) dropwise. This mixture was stirred for 1 h at room temperature, 16.4 mL of 1M HCl in ethyl ether was added, the solution was allowed to stir for 30 min, and the solvents removed on a rotary evaporator. The resulting residue was partitioned between 3:1 methylene chloride:etrahydofuran and water, the pH was adjusted to 10 with saturated sodium bicarbonate, and the aqueous layer was saturated with sodium chloride and extracted several times with 3:1 methylene chloride:tetrahydofuran. The combined organic layers were dried over sodium sulfate and the solvent removed. This material was purified by flash chromatography on a silica gel column which was prepared by slurry packing with chloroform. The impure compounds were loaded on the column as a chloroform solution. Elution proceeded with neat chloroform followed by 3% methanol up to 10% methanol in chloroform as needed to elute the desired compounds. Product fractions were combined and the solvent was removed on a rotary evaporator. This material was dissolved in a minimum of hot ethyl acetate and allowed to crystallize. Crystalline material was isolated by filtration followed by washing with a small amount of ice-cold ethyl acetate and used directly in the next step after drying overnight in a vacuum oven.

**Introduction of Diversity Element R₃ into Structure 7**. The appropriate pure diamine 6, produced in the previous step (0.05 mmol x the number of derivatives being prepared), was dissolved in THF (2 mL x the number of derivatives being prepared) and to this was added TEA (0.1 mmol x the number of derivatives being prepared). Then, into prelabeled, 20-mL scintillation vials containing a stir bar was added one of the chosen carboxylic acids (0.05 mmol). To this was added the appropriate fraction of the diamine/TEA/THF mixture followed by 50 µL of a 1M solution of BOP reagent in dimethylformamide (DMF). The vial was then capped with a telfon-lined lid and stirred for 1 h at room temperature. After this time, 4 mL of ethyl ether and 2 mL of water were added to the vial. After shaking and allowing the layers to settle, the aqueous layer was withdrawn with a pipette. Next, 2 mL of saturated sodium bicarbonate solution was added and the procedure repeated. This was followed by a similar wash with saturated sodium chloride solution. Sodium sulfate was added to the vial, and after drying, the mixture was pipetted into a preweighed, prelabeled 20-mL scintillation vial via a 6-in Pasteur pipette containing a small cotton plug. Following this, 2 mL of chloroform was added to the drying agent and the vial shaken after which the chloroform rinse was filtered as above. The collecting vials were placed under a nitrogen outlet and allowed to evaporate. Once the solvent was removed, the vials were placed in a high vacuum desiccator and allowed to remain overnight. The vials were reweighed, and the crude yield determined by difference. Since pilot studies showed that the BOP-coupling reaction produced very clean samples, the products were used without further purification, and the purity was taken to be 100%.

Prior to screening, all compounds were diluted to a concentration of 10 mM in dimethylsulfoxide (DMSO). Dilution was accomplished by determining the mean mmol/vial for each batch of 20 reactions using an Excel 3.0 spreadsheet. Weights deviating from the mean by >±10% were grouped into a second and third set above and below the mean. These were also averaged within the same parameters. Any compounds not falling within the above sets were diluted individually according to their weight This procedure permitted sample dilution to be accomplished using a minimum number of different volume deliveries of DMSO. Once diluted to 10 mM, 1-mL samples from each vial were withdrawn and placed in rows A and E (one compound/well) of a 1 mL x 96-well polypropylene microtiter plate. Serial dilution was then performed using Matrix multichannel pipettors which provided a 1-mM solution in rows B and F and a 0.1-mM solution in rows C and G. Once all of the compounds were transferred to plates and diluted to the proper concentration, the plates were placed in the refrigerator prior to assay.

### N-(2'-Aminoethyl)-(3R,4R)-dimethyl-4-(3-hydroxyphenyl)piperidine (6a).

Prepared from N-(tert-butoxy)-glycine and (+)-(3R,4R)-dimethyl-4-(3-hydroxyphenyl)piperidine according to the general procedure in 15% yield:¹ H NMR (MeOH-d4) δ 7.13-7.062 (t, 1H, *J*=8.1 Hz), 6.77-6.74 (m, 2H), 6.59-6.55 (m, 1H), 3.31-3.29 (m, 1H), 2.83-2.70 (m, 3H), 2.5 (d, 2H, *J* = 3.1 Hz), 2.46-2.27 (m, 3H), 2.00 (s, 1H), 1.6(d, 2H, *J* = 3.1 Hz), 1.68 (d, 1H, *J* =13.7 Hz), 1.29 (s, 3H), 0.89 (d, 3H, *J* = 7.0 Hz); ¹³C NMR (MeOH-d4) δ 158.5, 152.9, 130.0, 117.9, 113.9, 113.3, 61.6, 57.1, 51.5, 40.2, 39.5, 39.1, 32.0, 28.2, 16.7. MS (electrospray) M + 1 = 249. Calculated = 249.

**N-(2'-Methylaminoethyl)-(3R,4R)-dimethyl-4-(3-hydroxyphenyl)piperidine** (**6b**). Prepared from N-(tert-butoxy)-sarcosine and (+)-(3R,4R)-dimethyl-4-(3-hydroxyphenyl)piperidine according to the general procedure in 32% yield: 1 H NMR (MeOH-d4) δ 7.9 (t, 1H, *J* =7.7 Hz), 6.77 (d, 1H), 6.74 (s, 1H), 6.58 (d, 1H), 2.95-2.90 (m, 1H), 2.87-2.82 (m, 2H), 2.66 (dd, 1H), 2.61-2.55 (m, 2H), 2.54 (s, 3H), 2.52 (td, 1H), 2.37 (td, 1H), 2.03-2.00 (m, 1H), 1.69 (brd, 1H), 1.30 (s, 3H), 0.89 (d, 3H, *J* = 7.0 Hz); ¹³CNMR (MeOH-d4) δ 130.0, 118.0, 113.8, 113.3, 57.4, 56.7, 51.1, 48.2, 40.2, 39.4, 35.0, 31.9, 28.1, 16.6. MS (electrospray) M + 1 = 263. Calculated = 263.

### N-[(2'S)-Aminopropyl]-(3R,4R)-dimethyl-4-(3-hydroxyphenyl)piperidine (6c).

Prepared from N-(tert-butoxy)-L-alanine and (+)-(3R,4R)-dimethyl-4-(3-hydroxyphenyl)piperidine according to the general procedure in 56% yield: ¹H NMR (MeOH-d4) δ 7.11-7.08 (t, 1H, J=7.7), 6.78-6.76 (d, 1H), 6.74 (s, 1H), 6.59-6.57 (d, 1H), 2.953-2.902 (m, 1H), 2.874-2.826 (m, 2H), 2.676-2.647 (dd, 1H), 2.618-2.559 (m, 2H), 2.548 (s, 3H), 2.541-2.400 (td, 1H), 2.342-2.284 (td, 1H), 2.030-2.002 (m, 1H), 1.613-1.587 (brd, 1H), 1.303 (s, 3H), 0.800-0.786 (d, 3H, J = 7.0); ¹³C NMR (MeOH-d4) d 130.0, 118.0, 113.8, 113.3, 57.4, 56.7, 51.1, 48.2, 40.2, 39.4, 35.0, 31.9, 28.1, 16.6. MS (electrospray) M + 1 = 263. Calculated = 263.

**N-[(2'S)-(Methylamino)propyl]-(3R,4R)-dimethyl-4-(3-hydroxyphenyl)piperidine (6d)**. Prepared from N-(tert-butoxy)-N-methyl-L-alanine¹⁷ and (+)-(3R,4R)-dimethyl-4-(3-hydroxyphenyl)piperidine according to the general procedure in 33% yield: ¹HNMR (MeOH-d4) δ 7.18 (t, 1H, *J*=7.9 Hz), 6.76 (d, 1H), 6.73 (s, 1H), 6.57 (d, 1H), 2.72-2.64 (m, 2H), 2.61-2.47 (m, 3H), 2.36 (s, 3H), 2.34-2.20 (m, 3H), 2.00-1.99 (m, 1H), 1.56 (dd, 1H), 1.29 (s, 3H), 1.03 (d, 3H, *J*=6.2 Hz), 0.65 (d, 3H, *J*=6.9 Hz); ¹³C NMR (MeOH-d4) δ 158.4, 153.3, 130.1, 117.9, 113.7, 113.3, 65.1, 56.0, 52.9, 52.9, 40.0, 39.5, 33.7, 31.9, 28.0, 17.3, 16.7. MS (electrospray) M + 1 = 277. Calculated = 277.

**N-[(2'S)-Amino-3'-methylbutyl]-(3R,4R)-dimethyl-4-(3-hydroxyphenyl)piperidine (6e).** Prepared from N-(tert-butoxy)-L-valine and (+)-(3R,4R)-dimethyl-4-(3-hydroxyphenyl)piperidine according to the general procedure in 78% yield: ¹H NMR (MeOH-d4) δ 7.126-7.062 (t, 1H), 6.769-6.735 (m, 2H), 6.603-6.558 (m, 1H), 2.657-2.179 (m, 8H), 2.000 (brs, 1H), 1.583-1.502 (m, 2H), 1.294 (s, 3H), 0.978-0.912 (q, 6H), 0.789-0.761 (d, 3H); ¹³C NMR (MeOH-d4) δ 158.5, 153.3, 130.1, 117.8, 113.8, 113.3, 63.4, 55.8, 54.1, 53.3, 40.0, 39.5, 33.1, 31.9, 28.1, 19.6, 19.2, 16.8. MS (electrospray) M + 1 = 291. Calculated = 291.

**N-[(2'R)-Amino-3'-methylbutyl]-(3R,4R)-dimethyl-4-(3-hydroxyphenyl)piperidine (6f).** Prepared from N-(tert-butoxy)-D-valine and (+)-(3R,4R)-dimethyl-4-(3-hydroxyphenyl)piperidine according to the general procedure in 62% yield ¹H NMR (MeOH-d4) δ 7.11-7.08 (t, 1H), 6.78-6.76 (d, 1H), 6.74 (s, 1H), 6.59-6.57 (dd, 1H), 3.139-3.097 (m, 1H), 2.953 (brs, 1H), 2.894-2.865 (dd, 1H), 2.546-2.500 (m, 2H), 2.401-2.292 (m, 3H), 2.046-2.034 (brm, 1H), 1.894-1.827 (sext, 1H), 1.62-1.30 (m, 1H), 1.311 (s, 3H), 1.042-1.006 (dd, 6H), 0.834-0.820 (d, 3H); ¹³C NMR (MeOH-d4) δ 152.9, 130.1, 118.0, 113.8, 113.3, 59.8, 58.8, 55.2, 50.0, 40.4, 39.4, 31.6, 31.1, 28.0, 18.8, 18.5, 16.5. MS (electrospray) M + 1 = 291. Calculated = 291.

**N-[(2'S)-Amino-4'-methylpentyl]-(3R,4R)-dimethyl-4-(3-hydroxyphenyl)piperidine (6g).** Prepared from N-(tert-butoxy)-L-leucine and (+)-(3R,4R)-dimethyl-4-(3-hydroxyphenyl)piperidine according to the general procedure in 56% yield: ¹H NMR (MeOH-d4) δ 7.09 (t, 1H, *J*=7.9 Hz), 6.76 (d, 1 H, *J*=7.9 Hz), 6.73 (s, 1H), 6.57 (dd, 1H, *J*=2.2,7.9 Hz), 3.03-2.97 (m, 1H), 2.73 (d, 1H, *J*=11.2 Hz), 2.64 (d, 1H, *J*=11.1 Hz), 2.56 (td, 1H, *J*=2.5, 12.0 Hz), 2.48 (dd, 1H, *J*=2.7, 11.4 Hz), 2.33 (td, 1H, *J*=4.5, 12.7 Hz), 2.25 (dd, 1H. *J* = 3.6,12.4 Hz), 2.19-2.15 (m, 1H), 2.01-2.00 (m, 1H), 1.75 (sept, 1H, *J* =6.6 Hz), 1.56 (d, 1H. *J*=13.0 Hz), 1.29 (s, 3H), 1.27-1.15 (m, 2H). 0.94-0.91 (m, 6H), 0.07 (d, 3H, *J* =7.0 Hz); ¹³CNMR (MeOH-d4) δ 158.3, 153.3, 130:1, 117.9, 113.7, 113.2, 65.7, 56.0, 53.1, 46.5, 45.2, 40.0, 39.5, 31.9, 28.0, 25.8, 23.7, 22.6, 16.7. MS (electrospray) M + 1 = 305. Calculated = 305.

**N-[(2'S)-Amino-3'-methylpentyl]-(3R,4R)-dimethyl-4-(3-hydroxyphenyl)piperidine (6h).** Prepared from N-(tert-butoxy)-L-isoleucine and (+)-(3R,4R)-dimethyl-4-(3-hydroxyphenyl)piperidine according to the general procedure in 47% yield: ¹H NMR (MeOH-d4) δ 7.19 (t, 1H, *J* =7.9 Hz), 6.76 (d, 1H, *J*=8.1 Hz), 6.73-6.73 (m, 1H), 6.58-6.56 (dd, 1H,*J*=2.1, 7.9 Hz), 2.86-2.82 (m, 1H), 2.75-2.73 (m, 1H), 2.65-2.57 (m, 2H), 2.502-2.474 (dd, 1H, *J*=2.8,11.4 Hz), 2.40-2.23 (m, 3H), 2.02-2.00 (m, 1H), 1.50 (m, 2H), 1.46-1.41(m, 1H), 1.30 (s, 3H), 1.24-1.17 (m, 1H), 0.98-0.87(m, 6H), 0.78 (d, 3H, *J* =7.0 Hz); ¹³CNMR (MeOH-d4) δ 158.3,153.2, 130.1, 117.9, 113.7, 113.3, 61.9, 55.9, 53.1, 52.9, 49.0, 40.0, 39.5, 39.3, 31.9, 28.0, 26.6, 16.7, 15.1, 11.8. MS (electrospray) M + 1 = 305. Calculated = 305.

**N-[(2'S)-Amino-2'-cyclohexylethyl]-(3R,4R)-dimethyl-4-(3-hydroxyphenyl)piperidine (6i)**. Prepared from N-(tert-butoxy)-L-cyclohexylglycine and (+)-(3R,4R)-dimethyl-4-(3-hydroxyphenyl)piperidine according to the general procedure in 63% yield: ¹H NMR (MeOH-d4) δ 7.18 (t, 1H. *J*=7.9), 6.76 (d, 1H, *J*= 7.8 Hz), 6.75 (s, 1H), 6.57 (d, 1H, *J*=7.8 Hz), 2.74-2.70 (m, 2H), 2.63-2.55 (m, 2H), 2.47-2.45 (d, 1H, *J*=10.0 Hz), 2.48 (dd, 1H, *J*=2.9, 12.4 Hz), 2.36 (td, 1H, *J*=4.3, 12.6 Hz), 2.23 (t, 1H, *J*=11.6 Hz), 2.00 (m, 1H), 1.76-1.74 (m, 3H), 1.67 (d, 2H, *J*=11.9 Hz), 1.57 (d,1H. *J*=13.0 Hz), 1.39-1.16 (m, 7H), 1.09 (quint, 2H, *J*=12.4 Hz), 0.77 (d, 3H, *J*=6.8 Hz); ¹³C NMR (MeOH-d4) δ 158.3, 153.3, 130.1, 117.9, 113.7, 113.3, 162.6, 55.8, 53.4, 53.1, 42.9, 40.0, 39.5, 31.9, 30.9, 30.5, 30.2, 28.0, 27.6, 27.4, 16.7. MS (electrospray) M + 1 = 331. Calculated = 331.

**N-[(2'S)-Methylamino-2'-phenylethyl]-(3R,4R)-dimethyl-4-(3-hydroxyphenyl)-piperidine (6j).** Prepared from N-(tert-butoxy)-N-methyl-phenylglycine¹⁷ and (+)-(3R,4R)-dimethyl-4-(3-hydroxyphenyl)piperidine according to the general procedure in 44% yield: ¹H NMR (MeOH-d4) δ 7.34-7.22 (m, 5H), 7.13 (t, 1H, *J*=8.2 Hz), 6.80-6.77 (m, 2H), 6.61-6.69 (m, 1 H), 3.63 (dd, 1H, *J*=3.7*,* 12.6 Hz), 2.73 (brd, 2H, *J*=7.6 Hz), 2.64-2.52 (m, 3H), 2.38 (dd, 2H, *J*=3.6, 12.6 Hz), 2.25 (s, 3H), 2.04 (brd, 1H, *J*=6.3 Hz), 1.59 (d, 1H, J=12.9), 1.312 (s, 3H), 0.818-0.790 (d,3H, J=6.9); ¹³CNMR (MeOH-d4) δ 147.3, 142.5, 131.5, 119.5, 119.0, 118.0, 107.4, 103.2, 102.7, 68.7, 68.233, 67.7, 55.2, 52.9, 45.1, 42.5, 42.5, 29.2, 28.9, 24.2, 21.3, 17.7. MS (electrospray) M + 1 = 339. Calculated = 339.

**N-[(2'S)-Amino-3'-phenylpropyl]-(3R,4R)-dimethyl-4-(3-hydroxyphenyl)piperidine (6k).** Prepared from N-(tert-butoxy)-L-phenylalanine and (+)-(3R,4R)-dimethyl-4-(3-hydroxyphenyl)piperidine according to the general procedure in 44% yield: 1H NMR (MeOH-d4) δ 7.29 (t, 1H, *J*=7.4 Hz), 7.24-7.06 (m, 5H), 6.75-6.71 (m, 2H), 6.57-6.55(m, 1H), 3.86-3.84 (m, 5H), 3.22-3.94 (m, 1H), 2.83-2.69 (m, 2H), 2.63-2.39 (m, 5H), 2.35-2.24 (m, 2H), 1.97 (t, 1H, *J*=6.4 Hz), 1.54 (t, 1H, *J*=12.7 Hz), 1.27 (s, 3H), 0.74 (dd, 3H, *J*=6.95, 21.04 Hz); ¹³CNMR(MeOH-d4) δ 158.3, 153.3, 139.9, 130.6, 130.3, 130.0, 129.6, 129.2, 127.5, 127.1, 118.0, 117.9, 113.8, 113.7, 113.2, 65.0, 64.7, 61.0, 57.3, 56.1, 52.9, 52.1, 50.5, 49.5, 49.3, 49.2, 49.0, 48.8, 48.7, 48.5, 41.9, 41.5, 40.3, 40.0, 39.4, 31.9, 28.0, 16.7. MS (electrospray) M + 1 = 339. Calculated = 339.

**N-{(2'S)-[3-(4-Hydroxyphenyl)propanamido]-3'-methylbutyl}-(3R,4R)-dimethyl-4-(3-hydroxyphenyl)piperidine (8)**. Prepared from compound 6e and 3-(4-hydroxyphenyl)propionic acid according to the general procedure above in 74% yield and purified by silica gel chromatography. The hydrochloride salt was prepared using 1M HCl in ethyl ether/methanol and precipitated from ethyl acetate: mp 136-140 °C; 1H NMR (free base). CD30D δ 7.16 (t, *J*=7.94, Hz, 1 H), 7.04 (d, *J*=8.45 Hz, 2 H), 6.76 (d, *J*=7.78 Hz, 1 H), 6.72-6.69 (m, 2 H), 6.65 (dd, *J*=8.04, 1.76 Hz, 1 H), 4.02-3.98 (m, 1 H), 3.57 (d, *J*= 12.5 Hz, 1 H), 3.40 (ddd, *J* = 2.90, 11.6, 13.4 Hz, 2 H), 3.03 (dd, *J* = 10.5, 13.4 Hz, 1 Hz), 2.84 (t, 7.07 Hz, 2 H), 2.60 (t, 7.58 Hz, 2 H), 2.43 (dt, *J* = 13.21, 4.9 Hz, 1 H), 2.36-2.35 (m, 1 H), 1.85 (d, *J* = 14.5 Hz, 1 H), 1.87-1.76 (m, 1H), 1.42 (s, 3 H), 0.92 (t, *J* = 6.9 8 Hz, 6 H), 0.815 (d, *J* = 7.53, 3 H); ¹³C NMR, CD3OD δ 176.3, 159., 157.7, 153.8, 133.8, 131.3, 131.0, 118.9, 117.1, 114.6, 114.2, 62.0, 57.2, 53.2, 52.8, 40.9, 40.3, 33.1, 33.1, 32.5, 31.7, 28.8, 20.6, 18.9, 17.3. MS (electrospray) M + 1 = 439. Anal. (C₂₇H₃₉ClN₂O₃•1.5H₂O): C, H, N.

Compounds cited in Table 1 were removed from the library and purified by silica gel chromatography. The purity of the library sample was determined according to the formula [(mg isolated sample/mg crude mass sample) X 100].

**N-{(2'R)-[3-(4-Hydroxyphenyl)propanamido]-3'-methylbutyl}-(3R,4R)-dimethyl-4-(3-hydroxyphenyl)piperidine (9).** Prepared from compound **6f** and 3-(4-hydroxyphenyl)propionic acid according to the general procedure. Purity (85%); ¹H NMR (MeOH-d4) δ 7.83 (s, 3H), 7.13-7.00 (m, 3H), 6.77-6.67 (m, 4H), 6.61-6.57 (m, 1H), 3.96-3.89 (m, 1H), 2.86-2.78 (m, 3M, 2.62-2.58 (m, 1H), 2.48 (d, 3H, J=8.0 Hz), 2.36-2.14 (m, 4H), 1.94 (brd, 1H, J=6.3 Hz), 1.76 (sept, 1H, J=5.5 Hz), 1.51 (brd, 1H, J=11.0 Hz), 1.26 (s, 3H), 0.84-0.74 (m, 9H). MS (electrospray) M + 1= 439. Calculated = 439.

**N-{(2'S)-[(3-Phenylpropanamido)-3'-methyl]butyl}-(3R,4R)-dimethyl-4-(3-hydroxyphenyl)piperidine (10).** Prepared from compound 6e and 3-phenylpropionic acid according to the general procedure. Purity (87%); ¹H NMR (MeOH-d4) δ 7.25-7.22 (m, 2H), 7.17-7.13 (m, 4H), 6.82 (s, 1H), 6.76 (d, 1H, J=7.8 Hz), 6.70-6.68 (m, 1H), 5.74 (s, 1H), 4.02-3.97 (m, 1H), 2.99-2.87 (m, 2H), 2.74-2.69 (m, 1H), 2.64 (brd, 1H, J=1.3 Hz, 2.57-2.40 (m, 6H), 2.27-2.21 (m, 2H), 2.17 (s, 3H), 1.92=1.87 (m, 2H), 1.56 (d, 1H, 3=13.0 Hz), 1.28 (s, 3H), 0.81 (t, 6H, J=6.8 Hz), 0.69 (d, 3H, J=6.8 Hz). MS (electrospray) M + 1 = 423. Calculated = 423.

**N-{(2'S)-[3-(3-Hydroxyphenyl)propanamido]-3'-methylbutyl}-(3R,4R)-dimethyl-4-(3-hydroxyphenyl)piperidine (11).** Prepared from compound **6e** and 3-(3-hydroxyphenyl)propionic acid according to the general procedure. Purity (84%); ¹HNMR (MeOH-d4) δ 7.24-7.23 (m, 1H), 7.13-7.03 (m, 3H), 6.76-6.57 (m, 5H), 3.32-3.29 (m, 4H), 2.85-2.17 (m, 8H), 1.97 (brs, 1H), 1.75-1.73 (m, 1H), 1.57 (brd, 1H, J=12.3 Hz), 1.28 (s, 3H) 0.863 (t, 6H, J=6.5 Hz), 0.72 (d, 3H, J=7.0). MS (electrospray) M + I = 439. Calculated = 439.

**N-{(2'S)-[3-(2-Hydroxyphenyl)propanamido]-3'-methylbutyl}-(3R,4R)-dimethyl-4-(3-hydroxyphenyl)piperidine (12).** Prepared from compound **6e** and 3-(2-hydroxyphenyl)propionic acid according to the general procedure. Purity (**85%**); ¹H NMR (CDC13-d) δ 7.04-6.82 (m, 3H), 6.66-6.65 (m, 2H), 6.48-6.39 (m, 3H), 3.97-3.94 (m, 1H), 2.87-2.84 (m, 2H), 2.76 (d, 1H, J=11 Hz), 2.56-2.22 (m, 8H), 1.94-1.93 (brm, 1H), 1.80 (sextet, 1H, J=6.9 Hz), 1.52 (d, 1H, J=13.3 Hz), 1.26 (s, 3H), 0.84 (dd, 6H, J=13.1 Hz), 0.75 (d, 3H, J=6.9 Hz). MS (electrospray) M + 1 = 439. Calculated = 439.

**N-{(2'S)-[(4-Hydroxyphenyl)acetamido]-3'-methylbutyl}-(3R,4R)-dimethyl-4-(3-hydroxyphenyl)piperidine (13).** Prepared from compound **6e** and 4-hydroxyphenylacetic acid according to the general procedure. Purity (88%); ¹H NMR (MeOH-d4) δ 7.14-7.06 (m, 3H), 6.67-6.69 (m, 4H), 6.58 (d, 1H, J=8.1 Hz), 3.95-3.92 (m, 1H), 3.32-3.30 (m, 2H), 2.70-2.60 (m, 1H), 2.56-2.47 (m, 1H), 2.41-2.15 (m, 6H), 1.90 (brs, 1H), 1.81-1.74 (m, 1H), 1.51 (d, 2H, J=12.5 Hz), 1.25 (s, 3H), 0.86 (t, 6H, J=6.7 Hz), 0.67 (d, 3H, J=6.9 Hz). MS (electrospray) M + 1 = 425. Calculated = 425.

**N-{(2'S)-[*trans*-3-(4-Hydroxyphenyl)acrylamido]-3'-methylbutyl}-(3R,4R)-dimethyl-4-(3-hydroxyphenyl)piperidine (14)**. Prepared from compound **6e** and *trans*-3-(4-hydroxyphenyl)cinnamic acid according to the general procedure. Purity (85%); ¹H NMR (MeOH-d4) δ 7.25-7.37 (m, 3H), 7.11-7.04 (m, 1H), 6.79-6.72 (m, 4H), 6.56 (d, 1H, J=9.5 Hz), 6.47 (d, 1H, J=12.7 Hz), 4.10 (m, 1H), 2.80 (m, 1H), 2.64 (m, 1H), 2.54-2.26 (m, 5H), 1.95 (m, 2H), 1.56 (d, 1H, J=13.1), 1.28 (s, 3H), 0.94 (t, 6H, J=6.8 Hz), 0.70 (d, 3H, J=6.9) MS (electrospray) M + 1 = 437. Calculated = 437.

**N-{(2'S)-[3-(4-Fluorophenyl)propanamido]-3'-methylbutyl}-(3R,4R)-dimethyl-4-(3-hydroxyphenyl)piperidine (15).** Prepared from compound 6e and 3-(4-fluorophenyl)propionic acid according to the general procedure. Purity (89%); ¹H NMR (MeOH-d4) δ 7.23-7.17 (m, 2H), 7.69 (t, 1H, J=8.0 Hz), 6.99-6.92 (m, 2H), 6.76-6.73.(m, 2H), 6.60-6.54 (m, 1H), 3.96-3.90 (m, 1H), 2.88 (t, 2H, J=7.7), 2.76 (d, 1H, J=10.3 Hz), 2.65-2.32 (m, 8H), 1.97 (brs, 1H), 1.73-1.69 (m, 1H), 1.54 (d, 1H, J=12.1 Hz), 1.27 (s, 3H), 0.80 (t, 6H, J= 5.8 Hz), 0.71 (d, 3H, J=6.9 Hz). MS (electrospray) M + 1 = 441. Calculated = 441.

**N-{(2'S)-[3-(3,4-Dihydroxyphenyl)propanamido]-3'-methylbutyl}-(3R,4R)-dimethyl-4-(3-hydroxyphenyl)piperidine (16).** Prepared from compound 6e and 3-(3,4-dihydroxyphenyl)propionic acid according to the general procedure. Purity (78%); ¹HNMR (MeOH-d4) δ 7.09 (t, 1H, J=7.9 Hz), 6.76-6.73 (m, 2H), 6.67-6.49 (m, 4H), 3.92 (brs, 1H), 2.74 (t, 3H, J=7.6 Hz), 2.63-2.59 (m, 1H), 2.51-2.15 (m, 7H), 1.94 (brs, 1H), 1.75-1.70 (m, 1H), 1.55 (d, 1H, J=12.1Hz), 1.27 (s, 3H), 0.82 (t;6H, J=6.4 Hz), 0.71 (d, 3H, J=6.9 Hz). MS (electrospray) M + 1 = 455. Calculated = 455.

**N-{(2'S)-[3-(3-Methoxy4-hydrolyphenyl)propanamido]-3'-methylbutyl}-(3R,4R)-dimethyl-4-(3-hydroxyphenyl)piperidine (17).** Prepared from compound 6e and 3-(3-methoxy-4-hydroxyphenyl)propionic acid according to the general procedure. Purity (87%); ¹H NMR (MeOH-d4) d 7.15 (t, 1H, J=7.7 Hz), 6.81-6.76 (m, 3H), 6.67 (d, 3H, J=3.3 Hz), 3.98 (brm, 1H), 3.80 (s, 3H), 2.86-2.69 (m, 3H), 2.53-2.22 (m, 8H), 1.89 (brs, 2H), 1.55 (d, 1H, J=12.0 Hz), 1.27 (s, 3H), 0.82 (dd, 6H, J=6.6, 3.2 Hz), 0.67 (d, 3H, J=6.9 Hz). MS (electrospray) M + 1= 469. Calculated = 469.

**N-{(2'S)-[3-(3-Methoxyphenyl)propanamido]-3'-methylbutyl}-(3R,4R)-dimethy]4-(3-hydroxyphenyl)piperidine (18).** Prepared from compound 6e and 3-(3-methoxyphenyl)propionic acid according to the general procedure. Purity (88%); ¹H NMR (MeOH-d4) δ 7.30-7.12 (m, 4H), 6.9-6.8 (m, 4H), 3.95 (brs, 1H), 3.76 (s, 3H), 2.96 (d, 2H, J=6.8 Hz), 2.86-2.72 (m, 5H), 2.65-2.61 (m, 1H), 2.56-2.14 (m, 7H), 1.91(brs, 1H)_{.} 1.73-1.71 (m, 1H), 1.52 (d, 1H, J=13.0 Hz), 1.26 (s, 3H), 0.81 (t, 6H, J=6.7 Hz), 0.67 (d, 3H, J=6.9 Hz). MS (electrospray) M + 1 = 453. Calculated = 453.

**N-{2'-[3-(4-Hydroxyphenyl)propanamido]ethyl}-(3R,4R)-dimethyl-4-(3-hydroxyphenyl)piperidine (19).** Prepared from compound 6a and 3-(4-hydroxyphenyl)propionic acid according to the general procedure. Purity (82%); ¹H NMR (MeOH-d4) δ 7.13-6.99 (m, 3H), 6.79-6.67 (m, 4H), 6.59 (dd, 1H, J=7.3, 1.8 Hz), 3.32-3.25 (m, 3H), 2.83-2.77 (m, 3H), 2.58 (s, 2H), 2.46-2.15 (m, 6H), 1.98 (brs, 1H), 1.58 (brd, 1H, J=12.8 Hz), 1.29 (s, 3H), 0.76 (d, 3H, J=7.0 Hz). MS (electrospray) M + 1 = 397. Calculated = 397.

**N-{(2'S)-[3-(4-Hydroxyphenyl)propanamido]propyl}-(3R,4R)-dimethyl-4-(3-hydroxyphenyl)piperidine(20).** Prepared from compound 6c and 3-(4-hydroxyphenyl)propionic acid according to the general procedure. Purity (88%); ¹H NMR (MeOH-d4) δ 7.77 (s, 1 H), 7.08 (t, 1H, J=8.1 Hz), 6.98 (d, 2H, J=8.4 Hz), 6.74-6.67 (m, 4H), 6.7 (d, 1H, J=7.5 Hz), 4.03 (dd, 1H, J=6.4 Hz), 2.811-2.70 (m, 3H), 2.49 (s, 2H), 2.44-2.26 (m, 4H), 2.16 (td, 2H, J=3.7, 10.9 Hz), 1.92-1.89 (m, 1H), 1.50 (d, 1H, J=12.3 Hz), 1.23 (s, 3H), 1.04 (d, 3H, J=6.4 Hz), 0.71 (d, 3H, J=6.9 Hz). MS (electrospray) M + 1 = 411. Calculated = 411.

**N-{2'-[3-(4-Hydroxyphenyl)-N-methylpropanamido]ethyl}-(3R,4R)-dimethyl-4-(3-hydroxyphenyl)piperidine (21).** Prepared from compound 6b and 3-(4-hydroxyphenyl)propionic acid according to the general procedure. Purity (78%); ¹H NMR (MeOH-d4) δ 7.84 (s, 1H), 7.18-7.00 (m, 3H), 6.77-6.69 (m, 4H), 6.60 (d, 1H, J=8.1 Hz), 3.47-3.27 (m, 2H), 2.92-2.90 (m, 3H), 2.82-2.77 (m, 3H), 2.67-2.54 (m, 3H), 2.47-2.18 (m, 3H),1.96 (brs, 1H), 1.58-1.49 (m, 3H), 1.27 (d, 3H, J=2.91 Hz), 0.73 (t, 3H, J=6.5 Hz). MS (electrospray) M + I = 411. Calculated = 411.

**N-{(2'S)-[3-(4-Hydroxyphenyl)-N-methylpropanamido] propyl}-(3R,4R)-dimethyl-4-(3-hydroxyphenyl)piperidine (22).** Prepared from compound 6d and 3-(4-hydroxyphenyl)propionic acid according to the general procedure. Purity (89%); ¹HNMR (MeOH-d4) δ 7.09 (t, 1H, J=7.9Hz), 6.99 (d, 2H, J=8.2 Hz), 6.78-6.66 (m, 4H), 6.58-6.56 (m, 1H), 4.92-4.86 (m, 1H), 2.74 (s, 3H), 2.27-2.17 (m, 2H), 1.96-1.95 (brm, 1H), 1.55 (brd, 1H, J=14.3 Hz), 1.27 (s, 3H), 1.02 (d, 3H, J=6.7 Hz), 0.66 (d, 3H, J=6.9 Hz). MS (electrospray) M + 1 = 425. Calculated = 425.

**N-{(2'S)-[3-(4-Hydroxyphenyl)-N-methylpropanamido]-2'-phenylethyl}-(3R,4R)-dimethyl-4-(3-hydroxyphenyl)piperidine (23).** Prepared according to the general procedure using compound 6j and 3-(4-hydroxyphenyl)propionic acid according to the general procedure. Purity (86%); ¹H NMR (MeOH-d4) δ 7.69-7.66 (m, 1H), 7.45-7.42 (m, 1H), 7.32-6.97 (m, 7H), 6.76 (d, 1H, J=9.4 Hz), 6.73 (s, 1H), 6.66-6.64 (m, 1H), 6.59-6.57 (m, 1H), 6.05 (q, 1H, J=5.5 Hz), 3.00-2.71 (m, 9H), 2.65-2,63 (m, 2H), 2.29 (td, 1H, J=4.3, 8.4 Hz), 2.01-2.00 (brm, 1H), 1.59 (brd, 1H, J=12.0 Hz), 1.32-1.28 (m, 6H), 0.71 (d, 3H, J=6.9 Hz). MS (electrospray) M + 1 = 487. Calculated = 487.

**N-{(2'S)-[3-(4-Hydroxyphenyl)propanamido]-4'-methylpentyl}-(3R,4R)-dimethyl-4-(3-hydroxyphenyl)piperidine (24).** Prepared according to the general coupling procedure (though on a 3-mmol scale) using compound 6g and 3-(4-hydroxyphenyl)propionic acid in 85% yield. Crude products were then purified by silica gel chromatography using 10-25% methanol in chloroform: ¹H NMR (MeOH-d4) δ 7.85 (s, 1H), 7.26-7.06 (m, 6H), 6.97 (d, 2H, J=8.5Hz), 6.76-6.66 (m, 3H), 6.58 (d, 1H, J=7.2Hz), 4.27 (t, 1H, J=7.3Hz), 2.84-2.23 (m, 10H), 1.93 (brd, 1H, J=7.2Hz), 1.52 (d, 1H, J=12.0Hz), 1.25 (s, 3H), 1.05 (t, 1H, J=7.2Hz), 0.74 (d, 3H, J=6.9Hz); ¹³C NMR (MeOH-d4) δ 164.0, 147.5, 143.0, 142.6, 129.0,122.3, 119.9,119.6, 119.3, 118.5, 116.5, 107.3,105.5, 103.0, 1022, 51.6, 46.1, 40.8, 29.4, 29.3, 29.3, 28.7, 21.4, 21.0, 17.3. Anal. (C₂₈H₄₀N₂O₃):C, H, N.

**N-{(2'S)-[3-(4-Hydroxyphenyl)propanamido]-3'methylpentyl}-(3R,4R)-dimethyl-4-(3-hydroxyphenyl)piperidine (25).** Prepared according to the general procedure (though on a 3-mmol scale) using compound 6h and 3-(4-hydroxyphenyl)propionic acid in 81% yield. Crude products were then purified by silica gel chromatography using 10-25% methanol in chloroform: ¹H NMR (MeOH-d4) δ 7.59 (s, 1H), 6.90-6.76 (m, 3H), 6.52-6.45 (m, 3H), 6.36 (d, 1H, J=7.6Hz), 3.89 (brs, 1H), 2.56-2.54 (m, 3H), 2.39-1.95 (m, 9H), 1.70 (brs, 1H), 1.32-1.10 (m, 3H), 1.03 (s, 5H), 0.65-0.61 (m, 8H), 0.52-0.42 (m, 3H); ¹³C NMR (MeOH-d4) δ 163.8, 147.5, 146.0, 142.6, 122.2, 119.7, 119.4, 107.4, 105.5, 103.1, 102.6, 68.7, 53.7, 46.2, 41.0, 39.4, 39.1, 35.4, 33.4, 29.5, 28.9, 28.7, 21.5, 21.2, 17.5, 15.1, 13.3, 11.9. Anal. (C₂₈H₄₀N₂O₃): C, H, N.

**N-{(2'S)-[3-(4-Hydroxyphenyl)propanamido]-2'-cyclohexylethyl}-(3R,4R). dimethyl-4-(3-hydroxyphenyl)piperidine (26).** Prepared according to the general procedure (though on a 3-mmol scale) using compound 6i and 3-(4-hydroxyphenyl)propionic acid in 87% yield. Crude products were then purified by silica gel chromatography using 10-25% methanol in chloroform: ¹H NMR (MeOH-d4) δ 7.85-7.82 (m, 2H), 7.11-6.97 (m, 3H), 6.74-6.56 (m, 5H), 3.99-3.97 (m, 1H), 2.81-2.75 (m, 3H), 2.54 (m, 1H), 2.44-2.12 (m, 7H), 1.94 (brs, 1H), 1.54-1.26 (m, 3H), 1.25 (s, 3H), 1.02-0.68 (m, 10H); ¹³C NMR (MeOH-d4) δ 164.1, 147.5, 146.0, 142.6, 122.2, 119.7, 119.4, 107.3, 105.5, 103.1, 102.5, 68.7, 49.4, 45.5, 41.3, 40.9, 29.4, 28.8, 28.4, 21.5, 21.1, 17.4, 15.4. Anal. (C₃₀H₂₄N₂O₃): C, H, N.

**N-{(2'S)-[3-(4-Hydroxyphenyl)propanamido]-3'-phenylpropyl}-(3R,4R)-dimethyl-4-(3-hydroxyphenyl)piperidine (27).** Prepared according to the general procedure (though on a 3-mmol scale)using compound 6k and 3-(4-hydroxyphenyl)propionic acid in 82% yield. Crude products were then purified by silica gel chromatography using 10-25% methanol in chloroform: ¹HNMR (MeOH-d4) δ 7.88(s, 1H),7.12-7.00(m, 3H), 6.76-6.66 (m, 4H), 6.59-6.55 (m, 1H), 3.90 (m, 1H), 2.78 (q, 3H, J=7.0 Hz), 2.62-2.56 (m, 1H), 2.47-2.24 (m, 6H), 1.66-1.50 (m, 6H), 1.26 (s, 3H), 1.16-1.03 (m, 3H), 0.88-0.84 (m, 2H), 0.71 (d, 3H, J=6.9 Hz); ¹³C NMR (MeOH-d4) δ 164.1, 147.5, 146.0, 142.6, 122.1, 119.8, 119.4, 107.3, 105.5, 103.1, 102.6, 68.7, 50.1, 45.6, 41.2, 41.1, 31.7, 29.4, 28.8, 21.5, 21.1, 20.3, 18.4, 17.4, 16.8. Anal. (C₃₁H₂₈N₂O₃): C, H, N.

**Opioid Binding Assays.** Mu binding sites were labeled using [³H][D-Ala²-MePhe⁴,Gly-ol⁵]enkephalin ([³H]DAMGO) (2.0 nM, SA = 45.5 Ci/mmol), and delta binding sites were labeled using [³H][D-Ala²,D-Leu⁵]enkephalin(2.0 nM, SA = 47.5 Ci/mmol) using rat brain membranes prepared as described.⁴ Kappa-1 binding sites were labeled using [³H]U69,593 (2.0 nM, SA = 45.5 Ci/mmol) and guinea pig membranes pretreated with BIT and FIT to deplete the mu and delta binding sites.

[³H]DAMGO binding proceeded as follows: 12 x 75 mm polystyrene test tubes were prefilled with 100 µL of the test drug which was diluted in binding buffer (BB: 10 mM Tris-HCl, pH 7.4, containing 1 mg/mL BSA), followed by 50 µL of BB, and 100 µL of [³H]DAMGO in a protease inhibitor cocktail (10 mM Tris-HCl, pH 7.4, which contained bacitracin (1 mg/mL), bestatin (100 µg/mL), leupeptin (40 µg/mL), and chymostatin (20 µg/mL). Incubations were initiated by the addition of 750 µL of the prepared membrane preparation containing 0.2 mg/mL of protein and proceeded for 4 to 6 h at 25°C. The ligand was displaced by 10 concentrations of test drug, in triplicate, 2x. Nonspecific binding was determined using 20 µM levallorphan. Under these conditions, the K_{d} of [³H]DAMGO binding was 4.35nM. Brandel cell harvesters were used to filter the samples over Whatman GF/B filters, which were presoaked in wash-buffer (ice-cold 10 mM Tris-HCl, pH 7.4).

[³H][D-Ala²,D-Leu⁵]enkephalin binding proceeded as follows: 12 x 75 mm polystyrene test tubes were prefilled with 100 µL of the test drug which was diluted in BB, followed by 100 µL of a salt solution containing choline chloride (1M, final concentration of 100 mM), MnCl2 (30 mM, final concentration of 3.0 mM), and, to block mu sites, DAMGO (1000 nM, final concentration of 100 nM), followed by 50 µL of [³H][D-Ala²,D-Leu⁵]enkephalin in the protease inhibitor cocktail. Incubations were initiated by the addition of 750 µL of the prepared membrane preparation containing 0.41 mg/mL of protein and proceeded for 4 to 6 h at 25 °C. The ligand was displaced by 10 concentrations of test drug, in triplicate, 2x. Nonspecific binding was determined using 20 µM levallorphan. Under these conditions the K_{d} of [³H][D-Ala²,D-Leu⁵]enkephalin binding was 2.95 nM. Brandel cell harvesters were used to filter the samples over Whatman GF/B filters, which were presoaked in wash buffer (ice-cold 10 mM Tris-HCl, pH 7.4).

[³H]U69,593 binding proceeded as follows: 12 x 75 mm polystyrene test tubes were prefilled with 100 µL of the test drug which was diluted in BB, followed by 50 µL of BB, followed by 100 µL of [³H]U69,593 in the standard protease inhibitor cocktail with the addition of captopril (1 mg/mL in 0.1N acetic acid containing 10 mM 2-mercapto-ethanol to give a final concentration of 1 µg/mL). Incubations were initiated by the addition of 750 µL of the prepared membrane preparation containing 0.4 mg/mL of protein and proceeded for 4 to 6 h at 25 °C. The ligand was displaced by 10 concentrations of test drug, in triplicate, 2x. Nonspecific binding was determined using 1 µM U69,593. Under these conditions the K_{d} of [³H]U69,593 binding was 3.75 nM. Brandel cell harvesters were used to filter the samples over Whatman GF/B filters, which were presoaked in wash buffer (ice-cold 10 mM Tris-HCl, pH 7.4) containing 1% PEI.

For all three assays, the filtration step proceeded as follows: 4 mL of the wash buffer was added to the tubes, rapidly filtered and was followed by two additional wash cycles. The tritium retained on the filters was counted, after an overnight extraction into ICN Cytoscint cocktail, in a Taurus beta counter at 44% efficiency.

[³⁵S]-GTP-γ-S Binding Assay. Ten frozen guinea pig brains (Harlan Bioproducts for Science, Inc, Indianapolis, IN) were thawed, and the caudate putamen were dissected and homogenized in buffer A (3 mL/caudate) (Buffer A = 10 mM Tris-HCl, pH 7.4 at 4 °C containing 4 µg/mL leupeptin, 2 µg/mL chymostatin, 10 µg/mL bestatin, and 100 µg/mL bacitracin) using a polytron (Brinkman) at setting 6 until a uniform suspension was achieved. The homogenate was centrifuged at 30,000 x g for 10 min at 4°C and the supernatant discarded. The membrane pellets were washed by resuspension and centrifugation twice more with fresh buffer A, aliquotted into microfuge tubes, and centrifuged in a Tomy refrigerated microfuge (model MTX 150) at maximum speed for 10 min. The supernatants were discarded, and the pellets were stored at -80 °C until assayed.

For the [³⁵S]GTP-γ-S binding assay, all drug dilutions were made up in buffer B [50 mM TRIS-HCl, pH 7.7/0.1 % BSA]. Briefly, 12 x 75 mm polystyrene test tubes received the following additions: (a) 50 µL buffer B with or without an agonist, (b) 50 µL buffer B with or without 60 µM GTP-γ-S for nonspecific binding, (c) 50 µL buffer B with or without an antagonist, (d) 50 µL salt solution which contained in buffer B 0.3 nM [³⁵S]GTP-γ-S, 600 mM NaCl, 600 µM GDP, 6 mM dithiothreitol, 30 mM MgCl₂, and 6 mM EDTA, and (e) 100 µL membranes in buffer B to give a final concentration of 10 µg per tube. The final concentration of the reagents were 100 mM NaCl, 5 mM MgCl₂, 1 mM EDTA, 1mM dithiothreitol, 100 µM GDP, 0.1% BSA, 0.05-0.1 nM [³⁵S]GTP-γ-S, 500 nM or 10 µM agonists, and varying concentrations (at least 10 different concentrations) of antagonists. The reaction was initiated by the addition of membranes and terminated after 4 h by addition of 3 mL ice-cold (4°C) purified water (Milli-Q uv-Plus, Millipore) followed by rapid vacuum filtration through Whatman GF/B filters presoaked in purified water. The filters were then washed once with 5 mL ice-cold water. Bound radioactivity was counted by liquid scintillation spectroscopy using a Taurus (Micromedic) liquid scintillation counter at 98% efficiency after an overnight extraction in 5 mL Cytoscint scintillation fluid. Nonspecific binding was determined in the presence of 10 µM GTP-γ-S. Assays were performed in triplicate, and each experiment was performed at least 3x.

Data Analysis. The data of the two separate experiments (opioid binding assays) or three experiments ([³⁵S]-GTP-γ-S assay) were pooled and fit, using the nonlinear least-squares curve-fitting language MLAB-PC (Civilized Software, Bethesda, MD), to the two-parameter logistic equation⁶ for the best-fit estimates of the IC₅₀ and slope factor. The Kᵢ values were then determined using the equation: IC₅₀/1 + **(**[L]/K_{d}).

% Inhibition Data for Compounds of Formula (I) in a Kappa Receptor Assay

| R₁ | R₂ | R₃ (acid) | % Inhibition |
|---|---|---|---|
| H | H | PA5 | 13 |
| H | H | BA1 | 20 |
| H | H | BA2 | 20 |
| H | H | BA4 | 21 |
| H | H | BA6 | 32 |
| H | H | BA8 | 11 |
| H | H | BA9 | 24 |
| H | H | BA10 | 28 |
| H | H | BA12 | 6 |
| H | H | BA13 | 9 |
| H | H | BA14 | 11 |
| H | H | BA16 | 11 |
| H | H | BA22 | 2 |
| H | H | BA23 | 13 |
| H | H | BA24 | 2 |
| H | H | BA25 | 6 |
| H | H | AA2 | 1 |
| H | H | AA4 | 0 |
| H | H | PP1 | 9 |
| H | H | PP2 | 23 |
| H | H | PP3 | 17 |
| H | H | PP4 | 1 |
| H | H | PP5 | 8 |
| H | H | PP6 | 14 |
| H | H | PP12 | 29 |
| H | H | PP15 | 19 |
| H | H | FA1 | 13 |
| H | H | FA2 | 9 |
| H | H | FA3 | 50 |
| H | H | FA4 | 33 |
| H | H | FA5 | 39 |
| H | H | FA6 | 27 |
| H | H | FA7 | 29 |
| H | H | FA8 | 35 |
| H | H | FA9 | 33 |
| H | H | FA10 | 8 |
| H | H | HA1 | 20 |
| H | H | HA2 | 42 |
| H | H | HA3 | 9 |
| H | H | HA4 | 15 |
| H | H | HA5 | 20 |
| H | H | OA23 | 8 |
| H | H | PB1 | 37 |
| H | H | CA2 | 35 |
| H | H | CA10 | 23 |
| H | H | CA11 | 13 |
| H | H | CA12 | 15 |
| H | H | PA38 | 14 |
| H | H | CA19 | 10 |
| H | H | CA20 | 12 |
| H | H | CA22 | 19 |
| H | H | CA38 | 27 |
| H | H | PA9 | 18 |
| H | H | PA10 | 9 |
| H | H | PA13 | 25 |
| H | H | PA15 | 17 |
| H | H | PA18 | 16 |
| H | H | PA23 | 9 |
| H | H | PA27 | 18 |
| H | H | PA28 | 9 |
| H | H | PA29 | 10 |
| H | H | PA32 | 22 |
| H | H | PA3 | 20 |
| H | H | PA4 | 11 |
| H | H | PA7 | 9 |
| H | H | PA17 | 13 |
| H | H | PA22 | 19 |
| H | H | PA8 | 23 |
| H | H | NA1 | 16 |
| H | H | NA2 | 6 |
| H | H | NA3 | 13 |
| H | H | NA4 | 1 |
| H | H | NA5 | 10 |
| H | H | NA6 | 2 |
| H | H | NA7 | 2 |
| H | H | NA8 | 15 |
| H | H | NA9 | 26 |
| H | H | NA10 | 22 |
| H | H | NA11 | 15 |
| H | H | BA7 | 2 |
| H | H | PA38 | 5 |
| H | H | AA1 | 8 |
| H | H | AA2 | 6 |
| H | H | AA4 | 3 |
| H | H | PP6 | 11 |
| H | CH₃ | BA4 | 12 |
| H | CH₃ | BA10 | 13 |
| H | CH₃ | BA1 | 6 |
| H | CH₃ | CA1 | 9 |
| H | CH₃ | CA5 | 6 |
| H | CH₃ | PA37 | 5 |
| H | CH₃ | PA5 | 11 |
| H | CH₃ | PA14 | 0 |
| H | CH₃ | PA32 | 0 |
| H | CH₃ | PP2 | 0 |
| H | CH₃ | PP5 | 0 |
| H | CH₃ | PP6 | 0 |
| H | CH₃ | PP1 | 0 |
| H | CH₃ | PP7 | 0 |
| H | CH₃ | BA11 | 0 |
| H | CH₃ | CA4 0 | |
| αi-Pr | H | BA4 | 9 |
| αi-Pr | H | BA5 | 19 |
| αi-Pr | H | BA8 | 0 |
| αi-Pr | H | BA7 | 5 |
| αi-Pr | H | BA11 | 0 |
| αi-Pr | H | BA12 | 0 |
| αi-Pr | H | BA13 | 26 |
| αi-Pr | H | BA15 | 1 |
| αi-Pr | H | BA19 | 0 |
| αi-Pr | H | BA20 | 0 |
| αi-Pr | H | BA21 | 3 |
| αi-Pr | H | CA1 | 0 |
| αi-Pr | H | CA10 | 0 |
| αi-Pr | H | CA11 | 0 |
| αi-Pr | H | CA7 | 0 |
| αi-Pr | H | PA5 | 6 |
| αi-Pr | H | PA7 | 14 |
| αi-Pr | H | PA9 | 0 |
| αi-Pr | H | PA10 | 0 |
| αi-Pr | H | PA13 | 10 |
| αi-Pr | H | PA15 | 4 |
| αi-Pr | H | PA18 | 7 |
| αi-Pr | H | PA22 | 0 |
| αi-Pr | H | PA27 | 18 |
| αi-Pr | H | PA28 | 6 |
| αi-Pr | H | CA16 | 0 |
| αi-Pr | H | CA18 | 0 |
| αi-Pr | H | PA29 | 1 |
| αi-Pr | H | PP1 | 28 |
| αi-Pr | H | PP2 | 3 |
| αi-Pr | H | PP3 | 27 |
| αi-Pr | H | PP4 | 18 |
| αi-Pr | H | PP5 | 20 |
| αi-Pr | H | PP6 | 70 |
| αi-Pr | H | PP7 | 13 |
| αi-Pr | H | PP8 | 17 |
| αi-Pr | H | PP12 | 23 |
| αi-Pr | H | PP15 | 26 |
| αi-Pr | H | PP16 | 31 |
| αi-Pr | H | PP17 | 43 |
| αi-Pr | H | CA5 | 4 |
| αi-Pr | H | CA7 | 16 |
| αi-Pr | H | CA12 | 15 |
| αi-Pr | H | PA8 | 21 |
| αi-Pr | H | PA23 | 6 |
| αi-Pr | H | PA32 | 13 |
| αi-Pr | H | BA1 | 3 |
| αi-Pr | H | CA4 | 3 |
| αi-Pr | H | PA18 | 7 |
| αi-Pr | H | NA1 | 14 |
| αi-Pr | H | NA2 | 3 |
| αi-Pr | H | NA3 | 16 |
| αi-Pr | H | NA4 | 43 |
| αi-Pr | H | NA5 | 61 |
| αi-Pr | H | NA6 | 1 |
| αi-Pr | H | NA7 | 22 |
| αi-Pr | H | NA8 | 3 |
| αi-Pr | H | NA9 | 33 |
| αi-Pr | H | NA10 | 3 |
| αi-Pr | H | NA11 | 34 |
| αi-Pr | H | BA7 | 25 |
| αi-Pr | H | PA38 | 4 |
| αi-Pr | H | AA1 | 3 |
| αi-Pr | H | AA2 | 4 |
| αi-Pr | H | AA4 | 13 |
| αi-Pr | H | CA2 | 5 |
| αi-Pr | H | FA1 | 5 |
| αi-Pr | H | FA2 | 6 |
| αi-Pr | H | FA3 | 9 |
| αi-Pr | H | FA4 | 17 |
| αi-Pr | H | FA5 | 10 |
| αi-Pr | H | FA6 | 10 |
| αi-Pr | H | FA7 | 10 |
| αi-Pr | H | FA8 | 27 |
| αi-Pr | H | FA9 | 14 |
| αi-Pr | H | FA10 | 6 |
| αi-Pr | H | HA1 | 6 |
| αi-Pr | H | HA2 | 1 |
| αi-Pr | H | HA3 | 0 |
| αi-Pr | H | HA4 | 10 |
| αi-Pr | H | HA5 | 10 |
| αi-Pr | H | OA23 | 0 |
| αi-Pr | H | PB1 | 7 |
| αi-Pr | H | PA14 | 8 |
| βi-Pr | H | PP4 | 52 |
| βi-Pr | H | PP6 | 11 |
| βi-Pr | H | PP8 | 10 |
| βi-Pr | H | PP12 | 50 |
| βi-Pr | H | PP15 | 24 |
| βi-Pr | H | PP16 | 8 |
| βi-Pr | H | PP17 | 10 |
| βi-Pr | H | PP18 | 5 |
| αCH₃ | H | PP6 | 11 |
| αCH₃ | CH₃ | CA1 | 3 |
| αCH₃ | CH₃ | CA2 | 0 |
| αCH₃ | CH₃ | CA8 | 0 |
| αCH₃ | CH₃ | CA14 | 0 |
| αCH₃ | CH₃ | CA15 | 1 |
| αCH₃ | CH₃ | CA19 | 0 |
| αCH₃ | CH₃ | CA20 | 10 |
| αCH₃ | CH₃ | CA24 | 5 |
| αCH₃ | CH₃ | CA28 | 0 |
| αCH₃ | CH₃ | CA30 | 7 |
| αCH₃ | CH₃ | BA1 | 7 |
| αCH₃ | CH₃ | BA4 | 7 |
| αCH₃ | CH₃ | BA8 | 8 |
| αCH₃ | CH₃ | BA13 | 8 |
| αCH₃ | CH₃ | BA19 | 8 |
| αCH₃ | CH₃ | BA20 | 5 |
| αCH₃ | CH₃ | BA21 | 5 |
| αCH₃ | CH₃ | BA23 | 6 |
| αCH₃ | CH₃ | BA25 | 5 |
| αCH₃ | CH₃ | PA5 | 6 |
| αCH₃ | CH₃ | PA8 | 1 |
| αCH₃ | CH₃ | PA10 | 0 |
| αCH₃ | CH₃ | PA19 | 0 |
| αCH₃ | CH₃ | PA21 | 0 |
| αCH₃ | CH₃ | PA27 | 4 |
| αCH₃ | CH₃ | PA28 | 0 |
| αCH₃ | CH₃ | PA29 | 1 |
| αCH₃ | CH₃ | PA32 | 0 |
| αCH₃ | CH₃ | PA14 | 0 |
| αCH₃ | CH₃ | PP1 | 6 |
| αCH₃ | CH₃ | PP4 | 2 |
| αCH₃ | CH₃ | PP5 | 3 |
| αCH₃ | CH₃ | . PP7 | 1 |
| αCH₃ | CH₃ | PP8 | 0 |
| αCH₃ | CH₃ | PP10 | 5 |
| αCH₃ | CH₃ | BAL1 | 0 |
| αCH₃ | CH₃ | GAB1 | 0 |
| αCH₃ | CH₃ | INP1 | 0 |
| αCH₃ | CH₃ | CA13 | 1 |
| αCH₃ | CH₃ | PA17 | 0 |
| αCH₃ | CH₃ | PA9 | 10 |
| αCH₃ | CH₃ | BA24 | 2 |
| αCH₃ | CH₃ | PP2 | 5 |
| αCH₃ | CH₃ | PP3 | 1 |
| αCH₃ | CH₃ | PP6 | 1 |
| αCH₃ | CH₃ | PA20 | 4 |
| αPh | CH₃ | CA1 | 0 |
| αPh | CH₃ | CA4 | 0 |
| αPh | CH₃ | CA9 | 1 |
| αPh | CH₃ | CA14 | 0 |
| αPh | CH₃ | CA15 | 3 |
| αPh | CH₃ | CA19 | 0 |
| αPh | CH₃ | CA20 | 2 |
| αPh | CH₃ | BA1 | 3 |
| αPh | CH₃ | BA2 | 0 |
| αPh | CH₃ | BA4 | 0 |
| αPh | CH₃ | PA14 | 3 |
| αPh | CH₃ | PA19 | 0 |
| αPh | CH₃ | PP1 | 4 |
| αPh | CH₃ | PP2 | 4 |
| αPh | CH₃ | OA1 | 9 |
| αPh | CH₃ | OA3 | 4 |
| αPh | CH₃ | CA2 | 5 |
| αPh | CH₃ | BA21 | 7 |
| αPh | CH₃ | PP3 | 5 |
| αPh | CH₃ | GAB1 | 11 |
| αPh | CH₃ | BA8 | 4 |
| αPh | CH₃ | BA10 | 0 |
| αPh | CH₃ | BA15 | 15 |
| αPh | CH₃ | PA8 | 1 |
| αPh | CH₃ | PA9 | 0 |
| αPh | CH₃ | PA10 | 6 |
| αPh | CH₃ | PA20 | 6 |
| αPh | CH₃ | PA21 | 9 |
| αPh | CH₃ | PP6 | 7 |
| αPh | CH₃ | PP7 | 0 |
| αPh | CH₃ | PP8 | 0 |
| αPh | CH₃ | OA2 | 0 |

| **Amino Alkyl Acids** | | |
|---|---|---|
| AA 1 | 1-Piperidine Propionic Acid | 157.21 |
| AA 2 | 2-N,N-Dimethyl Glycine | 103.21 |
| AA 3 | 3-N,N-Dimethyl Amino Propionic Acid | |
| AA 4 | 4-N,N-Dimethyl Amino Butyric Acid | 167.64 |

| **Benzoic Acids** | | |
|---|---|---|
| BA 1 | Benzoic Acid | 122.12 |
| BA 2 | 2-Chlorobenzoic Acid | 156.57 |
| BA 3 | 2-Acetamidobenzoic Acid | 179.18 |
| BA 4 | 2-Phenoxybenzoic Acid | 214.22 |
| BA 6 | 3-Chlorobenzoic Acid | 156.57 |
| BA 8 | 3-Phenoxybenzoic Acid | 214.22 |
| BA 9 | 3-Hydroxybenzoic Acid | 138.12 |
| BA 10 | 4-Chlorobenzoic Acid | 156.57 |
| BA 7 | 4-Dimethylaminobenzoic Acid | 165.19 |
| BA 12 | 4-Dodecyloxybenzoic Acid | 306.45 |
| BA 13 | 4-Butoxybenzoic Acid | 212.69 |
| BA 14 | 4-Hydroxybenzoic Acid | 138.12 |
| BA 16 | 4-tert-butylbenzoic Acid | 178.23 |
| BA 18 | 4-Acetamidobenzoic Acid. | 179.18 |
| BA 19 | o-Anisic Acid | 152.15 |
| BA 20 | m-Anisic Acid | 152.15 |
| BA 21 | p-Anisic Acid | 152.15 |
| BA 22 | 2-Benzoylbenoic Acid | 226.23 |
| BA 23 | 2-Biphenylbenzoic Acid | 98.22 |
| BA 24 | 4-Biphenylbenzoic Acid | 98.22 |
| BA 25 | 3-Dimethylaminobenzoic Acid | 165.19 |
| BA 26 | 2-Fluorobenzoic Acid | 140.11 |
| BA27 | 3-Nitrobenzoic Acid | 167.12 |
| BA 28 | o-Tolylic Acid | 136.15 |
| BA 29 | m-Tolylic Acid | 136.15 |
| BA 30 | p-Tolylic Acid | 136.15 |
| BA 31 | 4-Fluoro-3-nitrobenzoic | 185.11 |
| BA 32 | 3,4-Dichlorobenzoic Acid | 191.01 |
| BA33 | 2-Hydroxy Benzoin acid | 138.12 |
| BA34 | 4-Chloro-3-Nitro Benzoic Acid | 201.57 |
| BA35 | 4-Flurobenzoic Acid | 140.11 |
| BA36 | 2-Nitrobenzoic acid | 167.12 |
| BA37 | 4-Nitrobenzoic acid | 167.12 |

| **Cinnamic Acids** | | |
|---|---|---|
| CA 1 | a-Methylcinnamic Acid | 162.19 |
| CA 2 | a-Phenylcinnamic Acid | 226.4 |
| CA 3 | 2-Bromo-4,5-dimethoxycinnamic Acid | 287.11 |
| CA 4 | 2-Chlorocinnamic Acid | 182.61 |
| CA 5 | 2,4-Dichlorocinnamic Acid | 217.05 |
| CA 6 | 3,4-Dihydroxycinnamic Acid | 180.16 |
| CA 7 | 2,4-Dimethoaycinnamic Acid | 208.21 |
| CA 8 | 3,5-Di-tert-butyl-4-hydroxycinnamic Acid | 276.37 |
| CA 9 | 3-Fluorocinnamic Acid | 166.15 |
| CA 10 | 2-Hydroxycinnamic Acid | 164.16 |
| CA11 | 3-Hydroxycinnamic Acid | 164.16 |
| CA 12 | 4-Hydroxycinnamic Acid | 164.16 |
| CA 13 | 2-Methoxycinnamic Acid | 178.19 |
| CA 14 | 3-Methoxycinnamic Acid | 178.19 |
| CA 15 | 4-Methoxycinnamic Acid | 178.19 |
| CA 16 | 2-Methylcinnamic Acid | 162.19 |
| CA 17 | 3-Methylcinnamic Acid | 162.19 |
| CA 18 | 4-Methylcinnamic Acid | 162.19 |
| CA 19 | 3-(1-Naphthyl)acrylic Acid | 224.46 |
| CA 20 | 4-Phenylcinnamic Acid | 224.26 |
| CA 21 | 3,4,5-Trimethoxycinnamic Acid | 238.24 |
| CA 22 | 4-Isopropylcinnamic acid | 190.24 |
| CA23 | 2,6-Dichloro | 218.063 |
| CA24 | 3-benzyloxy | 254.234 |
| CA25 | 2-bromo-4,5-dimethoxy | 287.12 |
| CA26 | 2-chloro-6-fluoro | 200.6 |
| CA27 | alpha-methyl-2,4,5-trimethoxy | 252.27 |
| CA28 | 2-n-hexyloxy | 250.22 |
| CA29 | 5-bromo-2-methoxy | 257.09 |
| CA30 | 2-benzyloxy | 254.234 |
| CA31 | 2,4,5-trimethoxy | 238.24 |
| CA32 | 2,6-difluoro | 184.14 |
| CA33 | 2-t-butylthio | 236.157 |
| CA34 | 2-chloro-5-nitro | 227.61 |
| CA35 | 2,3-dimethoxy | 208.21 |
| CA36 | 3,5-dit-buryl-4-hydroxy | 276.37 |
| CA37 | 2,5-dimethoxy | 208.22 |
| CA 38 | trans-Cinnamic Acid | 147 |
| CA39 | cis-Cinnamic Acid | 147 |

| | **Fatty Acids** | |
|---|---|---|
| FA 1 | Acetic Acid | 60.05 |
| FA 2 | Propionic Acid | 74.08 |
| FA 3 | Pivalic Acid | 102.13 |
| FA 4 | 1-Phenyl-l-cyclopentane Acid | 162.19 |
| FA 5 | 1-Phenyl-1-cyclopropane Acid | 190.24 |
| FA 6 | Isovaleric Acid | 102.13 |
| FA 7 | 4-Methylvaleric Acid | 116.16 |
| FA 8 | Cyclopentylacetic Acid | 128.17 |
| FA 9 | Cyclopentylcarboxylic Acid | 114.14 |
| FA 10 | trans-2-Phenyl-1-cyclopropyl CA | 162.19 |
| FA 11 | Cyclohexane carboxylic Acd | 128.17 |

| **Hydroxy Acids** | | |
|---|---|---|
| HA 1 | 2-Hydroxy-3-methyl butyric | 118.13 |
| HA 2 | 2-Hydroxy-2-methyl butyric | 118.13 |
| HA 3 | 3-Hydroxy butyric | 104.11 |
| HA 4 | 3-Hydroxy-4-trimethylamino butyric | 197.66 |
| HA 5 | 2-Phenyl-3-hydroxy propionic | 166.18 |

| **Nicotinic Acids** | | |
|---|---|---|
| NA 1 | 2(n-Amylthio)nicotinic Acid | 225.31 |
| NA 2 | 5-Bromonicotiriic Acid | 202.01 |
| NA 3 | 6-Chloronicotinic Acid | 157.56 |
| NA 4 | 2-Chloronicotinic Acid | 157.56 |
| NA 5 | 2-(Methylthio)nicotinic Acid | 169.2 |
| NA 6 | Nicotinic Acid | 123.11 |
| NA 7 | Picolinic Acid | 123.11 |
| NA 8 | 2-Pyridylacetic Acid HCl | 173.6 |
| NA 9 | 3-Pyridylacetic Acid HCl | 173.6 |
| NA 10 | 4-Pyridylacetic Acid HCl | 173.6 |
| NA 11 | 2-(Phenylthio)Nicotinic Acid | 231.27 |
| NA 12 | 2-Hydroxy-6-methyl Nicotinic | 153.14 |
| NA13 | 3-(3-pyridyl)acrylic acid | 149.15 |
| NA 14 | 3-(4-pyridyl)acrylic acid | 149.15 |

| **Propionic Acid** | | |
|---|---|---|
| PP1 | Phenyl Propionic | 150.18 |
| PP2 | 3,3-Diphenylpropionic Acid | 226.28 |
| PP3 | 3-Phenylbutyric Acid | 164.2 |
| PP4 | 3-(2-Hydroxyphenyl)propionic Acid | 166.18 |
| PP 5 | 3-(3-Hydrozyphenyl)propionic Acid | 166.18 |
| PP 6 | 3-(4-Hydroxyphenyl)propionic Acid | 166.18 |
| PP7 | 3-(3-Methoxyphenyl)propionic Acid | 180.2 |
| PP8 | 3-(4-Methoxyphenyl)propionic Acid | 180.2 |
| PP9 | 3-(3,4,5-Trimethoxyphenyl)propionic Acid | 240.26 |
| PP10 | 3-(2-Methoxyphenyl)propionic Acid | 180.2 |
| PP11 | 3-(2,5-Dimethoxyphenyl)propionic Acid | 210.24 |
| PP12 | 3-(2-Chlorophenyl)propionic Acid | 184.62 |
| PP13 | 3-(4-Aminophenyl)propionic Acid | 165.119 |
| PP14 | 3-(4-Fluorophenyl)propionic Acid | 168.17 |
| PP15 | 3-(3,4-Dihydroxyphenyl)propionic Acid | 182.18 |
| PP16 | 3-(3-Methoxy-4-hydroxyphenyl) | 196.2 |
| PP17 | 3-(3,5-dinotro-4-hydroxyphenyl) | 256.2 |
| PP18 | 3-(Pentaflurophenyl)propionic Acid | |
| PP19 | 3-(4-Bocaminophenyl)propionic Acid | 265 |
| PP21 | 2,2-Diphenylpropionic Acid | 226.28 |

| **Phenylacetic Acid** | | |
|---|---|---|
| PA 1 | 4-Aminophenylacetic Acid | 151.17 |
| PA 2 | 4-Biphenylacetic Acid | 288.55 |
| PA 3 | 2-Bromophenylacetic Acid | 215.05 |
| PA 4 | 4-Bromophenylacetic Acid | 215.05 |
| PA 5 | 4-(n-Butoxy)phenylacetic Acid | 208.26 |
| PA 7 | 3-Chloro-4-hydroxyphenylacetic Acid | 186.59 |
| PA 8 | 2-Chlorophenylacetic Acid | 170.6 |
| PA 9 | 3-Chlorophenylacetic Acid | 170.6 |
| PA 10 | 4-Chlorophenylacetic Acid | 170.6 |
| PA 11 | 2-Chloro-6-fluorophenylacetic Acid | 188.59 |
| PA 12 | 2,4-Dichlorophenylacetic Acid | 205.04 |
| PA 13 | 2,6-Dichlorophenylacetic Acid | 205.04 |
| PA 14 | 3,4-Dichlorophenylacetic Acid | 205.04 |
| PA 15 | 2,5-Dimethoxyphenylacetic Acid | 196.2 |
| PA 16 | 3,4-Dimethoxyphenylacetic Acid | 196.2 |
| PA 17 | 2,5-Dimethylphenylacetic Acid | 164.2 |
| PA 18 | 2,4-Dinitrophenylacetic Acid | 226.15 |
| PA 19 | 2-Fluorophenylacetic Acid | 154.14 |
| PA 20 | 3-Fluorophenylacetic Acid | 154.14 |
| PA 21 1 | 4-Fluorophenylaceiic Acid | 154.14 |
| PA 22 | 2-Hydroxyphenylacetic Acid | 152.15 |
| PA 23 | 4-Hydroxypbeoylacetic Acid | 152.15 |
| PA 24 | 2-Methoxypheirylacetic Acid | 166.18 |
| PA 25 | 3-Methoxyphenylacetic Acid | 166.18 |
| PA 26 | 4-Methoxyphenylacetic Acid | 166.18 |
| PA 27 | 2-Methylphenylacetic Acid | 150.18 |
| PA 28 | 3-Methylphenylacetic Acid | 150.18 |
| PA 29 | 4-Methylphenylacetic Acid | 150.18 |
| PA 30 | 2-Nitrophenylacetic Acid | 181.15 |
| PA 31 1 | 4-Nitrophenylacetic Acid | 181.15 |
| PA 32 | Phenylacetic Acid | 136.15 |
| PA 33 | 2-Trifluormethylophenylacetic Acid | 204.15 |
| PA 34 | 3-Trifluoromethylphenylacetic Acid | 204.15 |
| PA 35 | 3,4,5-Trimethozyphenylacetic Acid | 226.23 |
| PA 36 | 4-Ethoxyphenylacetic Acid | 180.22 |
| PA 37 | Mesitylacetic acid | 178.23 |
| PA 38 | 4-Dimethyl Amino PA | |
| PA 39 | 3-Hydroxyphenyl PA | |
| PA 40 | Diphenyl Acetic | |

### References

(1) Thomas, J.B.; Mascarella, S.W.; Rothman, R.B.; Partilla, J.S.; Xu, H.; McCullough, K.B.; Dersch, C.M.; Cantrell, B.E.; Zimmerman, D.M.; Carroll, F.I. Investigation of the N-substituent conformation governing potency and µ receptor subtype-selectivity in (+)-(3R,4R)-dimethyl-4-(3-hydroxyphenyl)piperidine opioid antagonists. J. Med. Chem. 1998, 41(11), 1980-1990.
(2) Mitch, C.H.; Leander, J.D.; Mendelsohn, L.G.; Shaw, W.N.; Wong, D.T.; Cantrell, B.E.; Johnson, B.G.; Reel, J.K.; Snoddy, J.D.; Takemori, A.E.; Zimmerman, D.M. 3,4-Dimethyl-4-(3-hydroxyphehyl)piperidines: Opioid antagonists with potent anorectant activity. J. Med. Chem. 1993, 36(20), 2842-2850.
(3) Xu, H.; Lu, Y.-F.; Partilla, J.S.; Brine, G.A.; Carroll, F.I.; Rice, K.C.; Lai, J.; Porreca, F.; Rothman, R.B. Opioid peptide receptor studies. 6. The 3-methylfentanyl congeners RTI-4614-4 and its enantiomers differ in efficacy, potency, and intrinsic efficacy as measured by stimulation of [35S]GTP-γ-S binding using cloned µ-opioid receptors. Analgesia 1997, 3, 35-42.
(4) Rothman, R.B.; Xu, H.; Seggel, M.; Jacobson, A.E.; Rice, K.C.; Brine, G.A.; Carroll, F.I. RTI-4614-4: an analog of (+)-cis-3-methylfentanyl with a 27,000-fold binding selectivity for mu versus delta opioid binding sites. Life Sci. 1991, 48, PL111-PL-116.
(5) Rothman, R.B.; Bykov, V.; de Costa, B.R.; Jacobson, A.E.; Rice, K.C.; Brady, L.S. Interaction of endogenous opioid peptides and other drugs with four kappa opioid binding sites in guinea pig brain. Peptides 1990, 11, 311-331.
(6) Rodbard, D.; Lenox, R.H.; Wray, H.L.; Ramseth, D. Statistical characterization of the random errors in the radioimmunoassay dose-response variable. Clin. Chem. 1976, 22, 350- 58.
(7) Takemori et al, J. Pharm. Exp. Ther.,1988, 246 (1), 255-258.

**Table 1. Results of Inhibition Screening of Selected Structural Isomers of Compound 8 Taken from the Library versus Kappa Opioid Selective Ligand [³H]U69,593**

| | | | | | | | | |
|---|---|---|---|---|---|---|---|---|
| 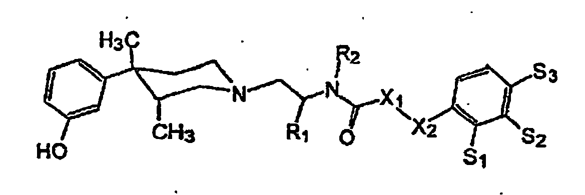 | | | | | | | | |
| compd | R1 | R2 | X1 | X2 | S₁ | S₂ | S₃ | % Inhibition at 100 nM |
| 8 | i-Pr | H | CH2 | CH2 | H | H | OH | 71 |
| 9 | i-Pr^{α} | H | CH2 | CH2 | H | H | OH | 11 |
| 10 | i-Pr | H | CH2 | CH2 | H | H | H | 28 |
| 11 | i-Pr | H | CH2 | CH2 | H | OH | H | 20 |
| 12 | i-Pr | H | CH2 | CH2 | OH | H | H | 25 |
| 13 | i-Pr | H | CH2 | -- | H | H | OH | 6 |
| 14 | i-Pr | H | CH2 | CH*^{b}* | H | H | OH | 15 |
| 15 | i-Pr | H | CH2 | CH2 | H | H | F | 26 |
| 16 | i-Pr | H | CH2 | CH2 | H | OH | OH | 31 |
| 17 | i-Pr | H | CH2 | CH2 | H | OCH3 | OH | 42 |
| 18 | i-Pr | H | CH2 | CH2 | H | H | OCH3 | 16 |
| 19 | H | H | CH2 | CH2 | H | H | OH | 11 |
| DMSO | | | | | | | | 4 |

| | | | | | | | | |
|---|---|---|---|---|---|---|---|---|
| *^{a}* The carbon to which the i-Pr group is attached has the opposite stereochemistry from that in 8. *^{b}* Trans double bond. | | | | | | | | |

**Table 2. Radioligand Binding Data for 8 and Related Compounds at Mu, Delta, and Kappa Opioid Receptor Assays**

| | | | | | | |
|---|---|---|---|---|---|---|
| 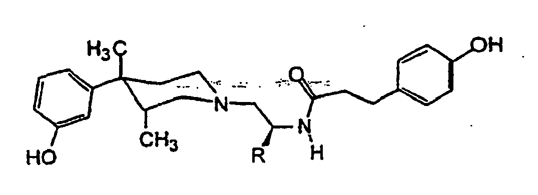 | | | | | | |

| compd | R | Ki (nM±SD) (-n_{H}) | | | µ/κ | δ/x |
|---|---|---|---|---|---|---|
| | | [³H]DAMGO | [³H]DADLE | [³H]U69, 593 | | |
| 8 | i-Pr | 393±13.3 (0.89±0.02) | >5700 | 6.91±0.55 (0.81±0.05) | 57 | >824 |
| 24 | i-Bu | 398±72.3 (0.91±0.16) | NA | 89.3±7.03 (0.78±0.05) | 4.5 | |
| 25 | sec-Bu | 421±30.5 (0.91±0.06) | NA | 8.84±0.30 (0.87±0.02) | 47 | |
| 26 | c-Hex | 234±25.2 (0.84±0.08) | NA | 83.1±5.7 (0.79±0.04) | 2.8 | |
| 27 | Benzyl | 9.6±:1.18 (0.89±0.09) | NA | 54.6±3.5 (0.86±0.04) | 0.17 | |
| 5a*^{a}* | | 0.74±0.05 (0.89±0.09) | 322±38.1 (0.75±0.09) | 122 ± 11.9 (0.52±0.07) | 0.006 | 2.6 |
| 1(nor-BNI)*^{b,c}* | | 47.2±3.3 | 42.9±11 | 0.28 ± 0.07 | 181 | 150 |
| naltrexone*^{b}* | | 1.39 ±0.40 (0.94±0.08) | 94.9±6.6 (1.01 ± 0.09) | 4.71±0.12 (1.05±0.08) | 0.30 | 20.1 |

| | | | | | | |
|---|---|---|---|---|---|---|
| *^{a}* Data taken from ref. 1. *^{b}* Data provided for reference; compound is not a derivative of 8. ^{c} Data taken from ref. 7. [³H]DAMGO, [³H]DPDPE, and [³H]U69593 were used as the radioligands for the mu, delta, and kappa assays, respectively. Guinea pig brain membranes were used. | | | | | | |

**Table 3. Inhibition by Antagonists of [³⁵S]GTPγS Binding in Guinea Pig Caudate Stimulated by DAMGO (µ), SNC80 (δ), and U69,593 (κ) Selective Opioid Agonists.**

| | Ki (nM±SD) (-n_{H})*^{a}* | | |
|---|---|---|---|
| Compd | DAMGO*^{b}* | SNC80*^{c}* | U69,593*^{d}* |
| 8 | 7.25 ± 0.52 | 450 ± 74.1 | 4.70 ± 0.56 |
| | (1.11 ± 0.08) | (1.05 ± 0.17) | (1.38 ± 0.19) |
| 5a*^{e}* | 0.039 ± 0.003 | 1.48 ± 0.094 | 1.04 ± 0.061 |
| | (1.06 ± 0.07) | (1.19 ± 0.08) | (1.07 ± 0.06) |
| 1, nor-BNI | 16.75 ± 1.47 | 10.14 ± 0.96 | 0.038 ± 0.005 |
| | (1.02 ± 0.09) | (1.18 ± 0.12) | (0.97 ± 0.12) |
| naltrexone | 0.93 ± 0.21 1 | 19.3 ± 2.25 | 2.05 ± 0.21 |
| | (1.03 ± 0.22) | (1.05 ± 0.17) | (1.38 ± 0.19) |

| | | | |
|---|---|---|---|
| *^{a}* See footnote a from Table 2. *^{b}* DAMGO [(D-Ala²,MePhe⁴,Gly-ol⁵)enkephalin]. | | | |

Agonist selective for mu opioid receptor. *^{c}* SNC-80 ([(+)-4-[(αR)-α-(2S,5R)-4-allyl-2,5-dimethyl-1-piperazinyl)-3-methoxybenzyl]-N,N-diethylbenzamide). Agonist selective for delta opioid receptor. *^{d}* U69,593 [(5α,7α,8β)-(-)-N-methyl-N-[7-(1-pyrrolidinyl)-1-oxaspiro[4,5]dec-8-yl]benzeneacetamide]. Agonist selective for kappa opioid receptor.,*^{e}* Data taken from ref. I.

### Analyses Appendix

**N-{(2'S)-[3-(4-Hydroxyphenyl)propanamido]-3'-methylbutyl}-(3R,4R)-dimethyl-4-(3-hydroxyphenyl)piperidine (8).**
   Anal. calcd for C₂₇H₃₉CIN₂O₃•1.5H₂O: C, 64.59, H, 8.43; N, 5.58. Found: C, 64.35; H, 8.12; N, 5.38.
**N-{(2'S)-[3-(4-Hydroxyphenyl)propanamido]-4'-methylpentyl}-(3R,4R)-dimethyl-4-(3-hydroxyphenyl)piperidine (24).**
   Anal. calcd for C₂₈H₄₀N₂O₃: C, 74.30, H, 8.91; N, 6.19. Found: C, 74.12; H, 9.22; N, 6.30.
**N-{(2'S)-[3-(4-Hydroxyphenyl)propanamido]-3'-methylpentyl}-(3R,4R)-dimethyl-4-(3-hydroxyphenyl)piperidine (25).**
   Anal. calcd for C₂₈H₄₀N₂O₃: C, 74.30, H, 8.91; N, 6.19. Found: C, 74.02; H, 9.20; N, 6.25.
**N-{(2'S)-[3-(4-Hydroxyphenyl)propanamido]-2'-cyclohexylethyl}-(3R,4R)-dimethyl-4-(3-hydroxyphenyl)piperidine (26).**
   Anal. calcd for C₃₀H₄₂N₂O₃: C, 75.28, H, 8.84; N, 5.85. Found: C, 75.18; H, 8.96; N, 5.97.
**N-{(2'S)-[3-(4-Hydroxyphenyl)propanamido]-3'-phenylpropyl}-(3R,4R)-dimethyl-4-(3-hydroxyphenyl)piperidine (27).**
   Anal. calcd for C₃₁H₃₈N₂O₃: C, 76.51, H, 7.87; N, 5.76. Found: C, 76.15; H, 7.99; N, 5.89.

### Results and Discussion

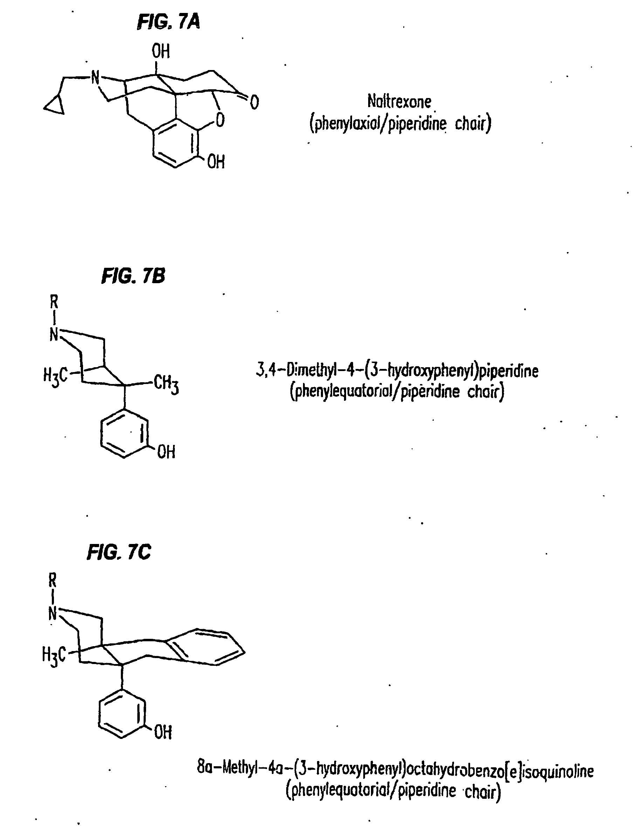

Both initial studies and work conducted in this laboratory have provided strong evidence that the antagonist activity of some N-substituted piperidine compounds is expressed via a phenyl equatorial/piperidine chair receptor-ligand interaction as illustrated in Figure 7b.² This stands in contrast to the phenylaxial/piperidine chair conformation exhibited by naltrexone (Figure 7a).

**Table 10. Affinities of the N-Substituted-3,4-dimethyl-(3'-hydroxyphenyl)piperidine Antagonists for the Mu and Kappa Opioid Receptors^{a}**

| Compd | Ki (nM) | |
|---|---|---|
| | µ [³H]Nal*^{b}* | κ [³H]EKC*^{c}* |
| **4a** | 80 | 833 |
| **4b** | 1.5 | 52 |
| **1b**, naltrexone | 0.56 | 3.9 |

| | | |
|---|---|---|
| *^{a}* Data taken from reference 2. *^{b}* [³H]Naloxone (µ receptor assay). *^{c}* [³H]Ethylketocyclazocine (κ receptor assay). | | |

**Table 11. Inhibition by Antagonists of [³⁵S]GTPγS Binding in Guinea Pig Caudate Stimulated by DAMGO (mu), SNC80 (delta), and U69,593 (kappa) Selective Opioid Agonists^{a}**

| Compd | Ki (nM±SD) | | |
|---|---|---|---|
| | µ (DAMGO)*^{a}* | δ (SNC80)*^{b}* | κ (U69,593)*^{c}* |
| **5b** | 21.2 ± 2.30 | 750 ± 85.9 | 105 ± 10.9 |
| **5c** | 0.338 ± 0.028 | 12.6 ± 1.01 | 1.34 ± 0.084 |
| **1b**, naltrexone | 0.930 ± 0.21 | 19.3 ± 2.25 | 2.05 ± 0.21 |

| | | | |
|---|---|---|---|
| *^{a}* DAMGO [(D-Ala²,MePhe⁴,Gly-ol⁵)enkephalin]. Agonist selective for mu opioid receptor. *^{b}*SNC-80 ([(+)-4-[(αR)-α-(2S,5R)-4-allyl-2,5-dimethyl-1-piperazinyl)-3-methoxybenzyl]-N,N-diethylbenzamide). Agonist selective for delta opioid receptor. Agonist selective for delta opioid receptor. *^{c}* U69,593 (trans-3,4-dichloro-N-methyl[2-(1-pyrrolidinyl)cyclohexyl]benzeneacetamide). Agonist selective for kappa opioid receptor. | | | |

### Appendix

Elemental Analysis

| Compd | Calcd. | | | Found | | |
|---|---|---|---|---|---|---|
| | C | H | N | C | H | N |
| **9** C₁₇H₂₅NO | 78.72 | 9.71 | 5.40 | 78.79 | 9.75 | 5.34 |
| **5b** C₁₆H₂₄ClNO | 68.19 | 8.53 | 4.91 | 68.25 | 8.53 | 5.03 |
| **5c** C₂₃H₃₀ClNO | 74.27 | 8.13 | 3.77 | 74.16 | 8.12 | 3.71 |

X-Ray Crystallographic Data and Analysis for Compound **5b**

**Table S1. Crystal data and structure refinement for 5b.**

| | | |
|---|---|---|
| Empirical formula | C₁₆ H₂₄ Cl N O | |
| Formula weight | 281.81 | |
| Temperature | 293(2) K | |
| Wavelength | 1.54178 Å | |
| Crystal system | Monoclinic | |
| Space group | P2(1)/c | |
| Unit cell dimensions | a =14.183(1) A | α= 90°. |
| | b = 9.996(1) A | β= 90.33(2)°. |
| | c = 11.126(1) A | γ = 90°. |
| Volume | 1577.5(2) Å³ | |
| Z | 4 | |
| Density (calculated) | 1.187 Mg/m³ | |
| Absorption coefficient | 2.072 mm⁻¹ | |
| F(000) | 608 | |
| Crystal size | 0.40 x 0.26 x 0.24 mm³ | |
| Theta range for data collection | 3.12 to 57.49° | |
| Index ranges | -15<=h<=3, -10<=k<=1, -12<=I<=12 | |
| Reflections collected | 2651 | |
| Independent reflections | 2154 [R(int) = 0.0312] | |
| Absorption correction | Integration | |
| Max. and min. transmission | 0.6449 and 0.5284 | |
| Refinement method | Full-matrix least-squares on F² | |
| Data / restraints / parameters | 2153 / 0 / 180 | |
| Goodness-of-fit on F² | 1.047 | |
| Final R indices [I>2sigma(I)] | R1 = 0.0472, wR2 = 0.1367 | |
| R indices (all data) | R1 = 0.0598, wR2 = 0.1493 | |
| Largest diff. peak and hole | 0.213 and -0.228 e.Å⁻³ | |

**Table S2. Atomic coordinates (x 10⁴) and equivalent isotropic displacement parameters (Å²x 10³) for 5b. U(eq) is defined as one third of the trace of the orthogonalized U^{ij} tensor.**

| | x | y | z | U(eq) |
|---|---|---|---|---|
| Cl(1) | 6176(1) | 534(1) | 8837(1) | 56(1) |
| C(1) | 6136(2) | -4826(3) | 7545(2) | 44(1) |
| N(2) | 6354(2) | -5819(2) | 6563(2) | 45(1) |
| C(2) | 5733(3) | -7019(3) | 6587(3) | 64(1) |
| C(3) | 7374(2) | -6184(3) | 6510(3) | 54(1) |
| C(4) | 8010(2) | -4963(3) | 6522(3) | 49(1) |
| C(5) | 7760(2) | -3857(3) | 7430(2) | 40(1) |
| C(6) | 7948(2) | -4300(3) | 8752(2) | 48(1) |
| C(7) | 7345(2) | -5466(3) | 9198(3) | 58(1) |
| C(8) | 6319(2) | -5367(3) | 8813(2) | 56(1) |
| C(9) | 6690(2) | -3545(2) | 7321(2) | 39(1) |
| C(9A) | 6394(2) | -2839(3) | 6157(2) | 48(1) |
| C(10) | 8344(2) | -2595(3) | 7206(2) | 43(1) |
| C(11) | 8071(2) | -1398(3) | 7730(3) | 49(1) |
| O(12) | 8256(2) | 962(2) | 8052(3) | 79(1) |
| C(12) | 8549(2) | -214(3) | 7554(3) | 56(1) |
| C(13) | 9351(2) | -206(4) | 6853(3) | 68(1) |
| C(14) | 9646(2) | -1386(4) | 6351(3) | 72(1) |
| C(15) | 9160(2) | -2568(3) | 6503(3) | 60(1) |

**Table S3. Bond lengths [Å] and angles [°] for 5b.**

| | |
|---|---|
| C(1)-N(2) | 1.509(3) |
| C(1)-C(9) | 1.524(4) |
| C(1)-C(8) | 1.532(4) |
| N(2)-C(2) | 1.488(4) |
| N(2)-C(3) | 1.494(4) |
| C(3)-C(4) | 1.517(4) |
| C(4)-C(5) | 1.540(4) |
| C(5)-C(10) | 1.530(4) |
| C(5)-C(9) | 1.553(3) |
| C(5)-C(6) | 1.558(4) |
| C(6)-C(7) | 1.530(4) |
| C(7)-C(8) | 1.518(4) |
| C(9)-C(9A) | 1.533(3) |
| C(10)-C(11) | 1.387(4) |
| C(10)-C(15) | 1.401(4) |
| C(11)-C(12) | 1.378(4) |
| O(12)-C(12) | 1.365(4) |
| C(12)-C(13) | 1.383(5) |
| C(13)-C(14) | 1.371(5) |
| C(14)-C(15) | 1.379(5) |
| N(2)-C(1)-C(9) | 109.1(2) |
| N(2)-C(1)-C(8) | 113.6(2) |
| C(9)-C(1)-C(8) | 111.2(2) |
| C(2)N(2)-C(3) | 112.2(2) |
| C(2)-N(2)-C(1) | 113.1(2) |
| C(3)-N(2)-C(1) | 113.1(2) |
| N(2)-C(3)-C(4) | 112.3(2) |
| C(3)-C(4)-C(5) | 116.4(2) |
| C(10)-C(5)-C(4) | 111.0(02) |
| C(10)-C(5)-C(9) | 110.5(2) |
| C(4)-C(5)-C(9) | 108.8(2) |
| C(10)-C(5)-C(6) | 107.4(2) |
| C(4)-C(5)-C(6) | 112.1(2) |
| C(9)-C(5)-C(6) | 107.0(2) |
| C(7)-C(6)-C(5) | 115.5(2) |
| C(8)-C(7)-C(6) | 113.3(2) |
| C(7)-C(8)-C(1) | 116.1(2) |
| C(1)-C(9)-C(9A) | 112.7(2) |
| C(1)-C(9)-C(5) | 108.9(2) |
| C(9A)-C(9)-C(5) | 114.9(2) |
| C(11)-C(10)-C(15) | 116.8(3) |
| C(11)-C(10)-C(5) | 119.4(2) |
| C(15)-C(10)-C(5) | 123.8(2) |
| C(12)-C(11)-C(10) | 122.9(3) |
| O(12)-C(12)-C(11) | 122.1(3) |
| O(12)-C(12)-C(13) | 118.5(3) |
| C(11)-C(12)-C(13) | 119.4(3) |
| C(14)-C(13)-C(12) | 118.6(3) |
| C(13)-C(14)-C(15) | 122.2(3) |
| C(14)-C(15)-C(10) | 120.0(3) |

**Table S4. Anisotropic displacement parameters (Å²x 10³)for 5b. The anisotropic displacement factor exponent takes the form: -2π²[ h²a*²U¹¹ + ... + 2 h k a* b* U¹² ]**

| | U¹¹ | U²² | U³³ | U²³ | U¹³ | U¹² |
|---|---|---|---|---|---|---|
| CI(1) | 62(1) | 59(1) | 48(1) | 3(1) | -5(1) | 10(1) |
| C(1) | 47(2) | 39(2) | 45(2) | 1(1) | -1(1) | -1(1) |
| N(2) | 58(2) | 34(1) | 43(1) | 3(1) | -7(1) | -4(1) |
| C(2) | 88(2) | 42(2) | 62(2) | 5(2) | -8(2) | -21(2) |
| C(3) | 67(2) | 44(2) | 52(2) | -7(1) | -4(1) | 11(2) |
| C(4) | 52(2) | 47(2) | 50(2) | -7(1) | -1(1) | 9(1) |
| C(5) | 41(1) | 36(1) | 42(1) | -2(1) | -2(1) | 5(1) |
| C(6) | 50(2) | 48(2) | 45(2) | -1(1) | -9(1) | 7(1) |
| C(7) | 80(2) | 51(2) | 43(2) | 8(1) | -8(2) | -2(2) |
| C(8) | 70(2) | 53(2) | 45(2) | 4(1) | 4(1) | -16(2) |
| C(9) | 39(1) | 35(1) | 42(1) | 0(1) | -1(1) | -1(1) |
| C(9A) | 50(2) | 40(2) | 56(2) | 6(1) | -8(1) | 1(1) |
| C(10) | 36(1) | 48(2) | 45(2) | 2(1) | -2(1) | -2(1) |
| C(11) | 41(2) | 44(2) | 62(2) | -3(1) | 4(1) | -4(1) |
| O(12) | 73(2) | 40(1) | 124(2) | -6(1) | 8(2) | -10(1) |
| C(12) | 48(2) | 49(2) | 69(2) | 2(2) | -8(2) | -8(1) |
| C(13) | 54(2) | 65(2) | 85(2) | 7(2) | -2(2) | -22(2) |
| C(14) | 44(2) | 92(3) | 83(2) | -1(2) | 14(2) | -19(2) |
| C(15) | 45(2) | 68(2) | 68(2) | -9(2) | 5(2) | -2(2) |

**Table S5. Hydrogen coordinates ( x 10⁴) and isotropic displacement parameters (Å²x 10³) for 5b.**

| | x | y | z | U(eq) |
|---|---|---|---|---|
| H(1A) | 5464(2) | -4606(3) | 7484(2) | 52 |
| H(2) | 6227(18) | -5389(28) | 5805(27) | 48(8) |
| H(2A) | 5086(3) | -6741(3) | 6621(3) | 97 |
| H(2B) | 5881(3) | -7550(3) | 7282(3) | 97 |
| H(2C) | 5832(3) | -7540(3) | 5874(3) | 97 |
| H(3A) | 7489(2) | -6694(3) | 5783(3) | 65 |
| H(3B) | 7531(2) | -6749(3) | 7191(3) | 65 |
| H(4A) | 8004(2) | -4574(3) | 5723(3) | 59 |
| H(4B) | 8649(2) | -5258(3) | 6687(3) | 59 |
| H(6A) | 8607(2) | -4549(3) | 8829(2) | 57 |
| H(6B) | 7843(2) | -3538(3) | 9274(2) | 57 |
| H(7A) | 7605(2) | -6297(3) | 8894(3) | 70 |
| H(7B) | 7378(2) | -5497(3) | 10069(3) | 70 |
| H(8A) | 5994(2) | -4795(3) | 9381(2) | 67 |
| H(8B) | 6040(2) | -6250(3) | 8870(2) | 67 |
| H(9A) | 6538(2) | -2934(2) | 7981(2) | 46 |
| H(9AA) | 6762(2) | -2041(3) | 6057(2) | 73 |
| H(9AB) | 5738(2) | -2608(3) | 6196(2) | 73 |
| H(9AC) | 6496(2) | -3425(3) | 5487(2) | 73 |
| H(11A) | 7544(2) | -1394(3) | 8222(3) | 59 |
| H(12) | 7646(36) | 906(46) | 8364(40) | 110(15) |
| H(13A) | 9683(2) | 583(4) | 6724(3) | 82 |
| H(14A) | 10192(2) | -1389(4) | 5893(3) | 87 |
| H(15A) | 9374(2) | -3347(3) | 6139(3) | 72 |

This Example is described in Thomas et al, J. Med. Chem., V. 41, No. 21, 4143-4149 (1998) incorporated herein by reference, inclusive of the "Supporting Information Available" described at p. 4149.

Obviously, numerous modifications and variations of the present invention are possible in light of the above teachings. It is therefore to be understood that within the scope of the appended claims, the invention may be practiced otherwise than as specifically described herein.

All references cited above are incorporated into this application by reference in their entirety unless noted otherwise.

## Claims

1. A compound represented by formula (I) : 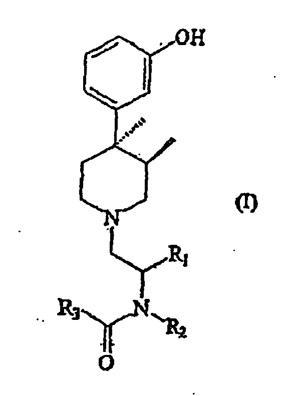 wherein
R₁ is hydrogen, a methyl group, an isopropyl-group, an isobutyl group, a cyclohexyl group, a secondary butyl group, a benzyl group, or a phenyl group ;
R₂ is hydrogen, or a methyl group ; and
R₃ is 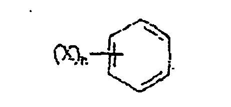 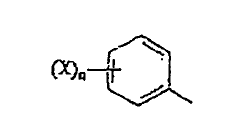 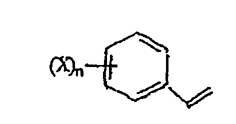 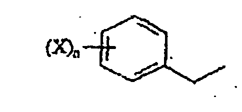 each X is independently, OH, OMethyl, OEthyl, OBenzyl, OnButyl, OPhenyl, Methyl, isopropyl, Butyl, Phenyl, N(CH3)2, C(O)NH₂, F, Cl,
n is 1 or 2 ;
or a pharmaceutically acceptable salt thereof.

2. The compound of claim 1, wherein
R₁ is hydrogen, a methyl group, an isopropyl group, an isobutyl group, a cyclohexyl group, a secondary butyl group, a benzyl group, or a phenyl group ;
R₂ is hydrogen, or a methyl group ;
n is 1 or 2.

3. The compound of claim 2, wherein
R₁ is hydrogen, a methyl group, an isopropyl group, an isobutyl group, or a secondary butyl group ;
n is 1, or 2.

4. The compound of claim 3, wherein
R₁ is hydrogen, a methyl group or an isopropyl group ;
R₂ is hydrogen or a methyl group ;
n is 1 or 2 and at least one X is -OH, -OCH₃ or -F.

5. The compound of claim 4, wherein at least one X is -OH.

6. A compound represented by the following formula : 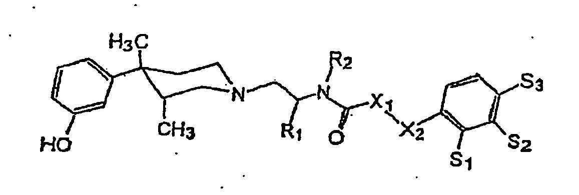 wherein R₁, R₂, X₁, X₂, S₁, S₂, S₃ are as defined in the following Table :
| Compd | R₁ | R₁ | X₁ | X₂ | S₁ | S₂ | S₃ |
|---|---|---|---|---|---|---|---|
| 8 | i-Pr | H | CH₂ | CH₂ | H | H | OH |
| 9 | i-Pr | H | CH₂ | CH₂ | H | H | OH |
| 10 | i-Pr | H | CH₂ | CH₂ | H | H | H |
| 11 | i-Pr | H | CH₂ | CH₂ | H | OH | H |
| 12 | i-Pr | H | CH₂ | CH₂ | OH | H | H |
| 13 | i-Pr | H | CH₂ | - | H | H | OOH |
| 14 | i-Pr | H | CH | CH | H | H | OH |
| 15 | i-Pr | H | CH₂ | CH₂ | H | H | F |
| 16 | i-Pr | H | CH₂ | CH₂ | H | OR | OH |
| 17 | i-Pr | H | CH₂ | CH₂ | H | OCH₃ | OH |
| 18 | i-Pr | H | CH₂ | CH₂ | H | H | OCH₃ |
| 19 | H | H | CH₂ | CH₂ | H | H | OH |
; and wherein in compound 9, the carbon to which the i-Pr group is attached has the opposite stereochemistry from that in compound 8 ; and in compound 14 the double bond linking X₁ and X₂ is a trans double bond.

7. The compound of formula (I) claim 1, wherein said compound is represented by the following formula : 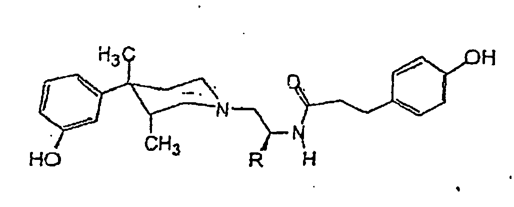 Wherein R is as defined in the following Table :
| Compd | R |
|---|---|
| 24 | i-Bu |
| 25 | sec-Bu |
| 26 | c-Hex |
| 27 | Benzyl |

## Patentansprüche

1. Verbindung, die durch Formel (I) wiedergegeben ist: 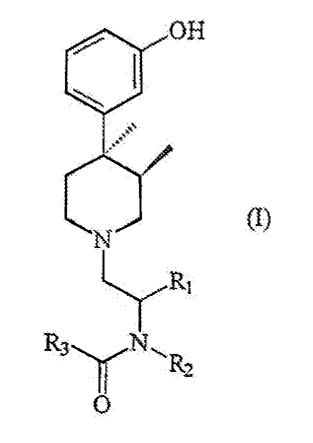 wobei
R₁ Wasserstoff, eine Methylgruppe, eine Isopropylgruppe, eine Isobutylgruppe, eine Cyclohexylgruppe, eine sekundäre Butylgruppe, eine Benzylgruppe oder eine Phenylgruppe ist;
R₂ Wasserstoff oder eine Methylgruppe ist; und
R₃ 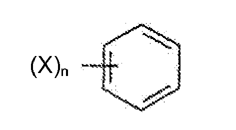 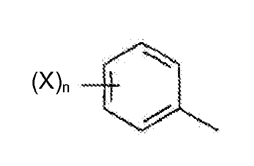 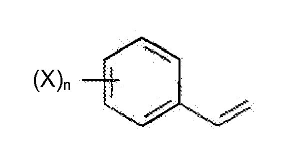 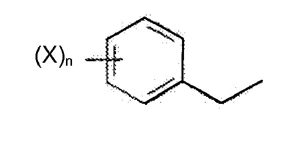 ist
jedes X unabhängig OH, OMethyl, OEthyl, OBenzyl, OnButyl, OPhenyl, Methyl, Isopropyl, Butyl, Phenyl, N(CH₃)₂, C(O)NH₂, F, CI ist;
n 1 oder 2 ist;
oder ein pharmazeutisch verträgliches Salz davon.

2. Verbindung nach Anspruch 1, wobei
R₁ Wasserstoff, eine Methylgruppe, eine Isopropylgruppe, eine Isobutylgruppe, eine Cyclohexylgruppe, eine sekundäre Butylgruppe, eine Benzylgruppe oder eine Phenylgruppe ist;
R₂ Wasserstoff oder eine Methylgruppe ist;
n 1 oder 2 ist.

3. Verbindung nach Anspruch 2, wobei
R₁ Wasserstoff, eine Methylgruppe, eine Isopropylgruppe, eine Isobutylgruppe oder eine sekundäre Butylgruppe ist;
n 1 oder 2 ist.

4. Verbindung nach Anspruch 3, wobei
R₁ Wasserstoff, eine Methylgruppe oder eine Isopropylgruppe ist;
R₂ Wasserstoff oder eine Methylgruppe ist;
n 1 oder 2 ist und wenigstens ein X -OH, -OCH₃ oder -F ist.

5. Verbindung nach Anspruch 4, wobei wenigstens ein X -OH ist.

6. Verbindung, die durch die folgende Formel wiedergegeben wird: 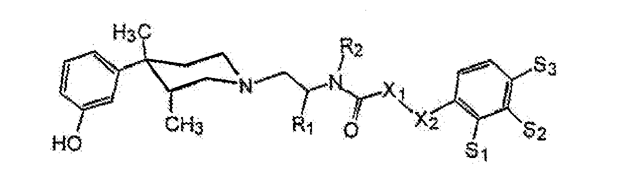 wobei R₁, R₂, X₁, X₂, S₁, S₂, S₃ wie in der folgenden Tabelle definiert sind:
| Verbindung | R₁ | R₂ | X₁ | X₂ | S₁ | S₂ | S₃ |
|---|---|---|---|---|---|---|---|
| 8 | i-Pr | H | CH₂ | CH₂ | H | H | OH |
| 9 | i-Pr | H | CH₂ | CH₂ | H | H | OH |
| 10 | i-Pr | H | CH₂ | CH₂ | H | H | H |
| 11 | i-Pr | H | CH₂ | CH₂ | H | OH | H |
| 12 | i-Pr | H | CH₂ | CH₂ | OH | H | H |
| 13 | i-Pr | H | CH₂ | - | H | H | OH |
| 14 | i-Pr | H | CH | CH | H | H | OH |
| 15 | i-Pr | H | CH₂ | CH₂ | H | H | F |
| 16 | i-Pr | H | CH₂ | CH₂ | H | OH | OH |
| 17 | i-Pr | H | CH₂ | CH₂ | H | OCH₃ | OH |
| 18 | i-Pr | H | CH₂ | CH₂ | H | H | OCH₃ |
| 19 | H | H | CH₂ | CH₂ | H | H | OH |
und wobei in Verbindung 9 der Kohlenstoff, an den die i-Pr-Gruppe gebunden ist, die entgegengesetzte Stereochemie von der in Verbindung 8 aufweist; und in Verbindung 14 die Doppelbindung, die X₁ und X₂ verbindet, eine trans-Doppelbindung ist.

7. Verbindung der Formel (I) nach Anspruch 1, wobei die Verbindung durch die folgende Formel wiedergegeben ist: 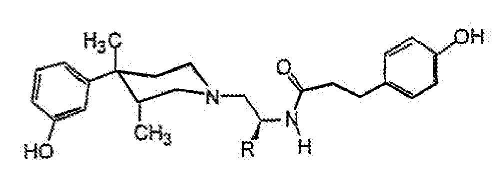 wobei R wie in der folgenden Tabelle definiert ist:
Tabelle:
| Verbindung | R |
|---|---|
| 24 | i-Bu |
| 25 | sec-Bu |
| 26 | c-Hex |
| 27 | Benzyl |

## Revendications

1. Composé représenté par la formule (I) : 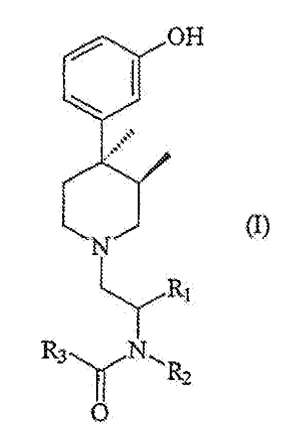 dans laquelle
R₁ est un hydrogène, un groupe méthyle, un groupe isopropyle, un groupe isobutyle, un groupe cyclohexyle, un groupe butyle secondaire, un groupe benzyle ou un groupe phényle ;
R₂ est un hydrogène ou un groupe méthyle ; et
R₃ est 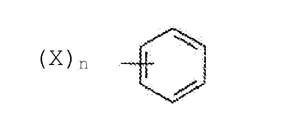 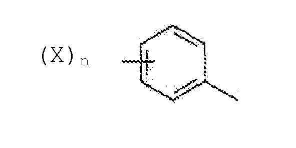 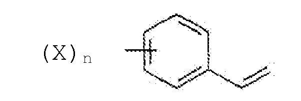 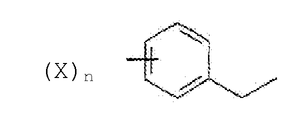 chaque X est indépendamment OH, OMéthyle, OEthyle, OBenzyle, OnButyle, OPhényle, méthyle, isopropyle, butyle, phényle, N(CH₃)₂, C(O)NH₂, F, Cl ;
n est 1 ou 2 ;
ou un sel pharmaceutiquement acceptable de celui-ci.

2. Composé selon la revendication 1, dans lequel
R₁ est un hydrogène, un groupe méthyle, un groupe isopropyle, un groupe isobutyle, un groupe cyclohexyle, un groupe butyle secondaire, un groupe benzyle ou un groupe phényle ;
R₂ est un hydrogène ou un groupe méthyle ;
n est 1 ou 2.

3. Composé selon la revendication 2, dans lequel
R₁ est un hydrogène, un groupe méthyle, un groupe isopropyle, un groupe isobutyle ou un groupe butyle secondaire ;
n est 1 ou 2.

4. Composé selon la revendication 3, dans lequel R₁ est un hydrogène, un groupe méthyle ou un groupe isopropyle ;
R₂ est un hydrogène ou un groupe méthyle ;
n est 1 ou 2 et au moins un X est -OH, -OCH₃ ou -F.

5. Composé selon la revendication 4, dans lequel au moins un X est -OH.

6. Composé représenté par la formule suivants : 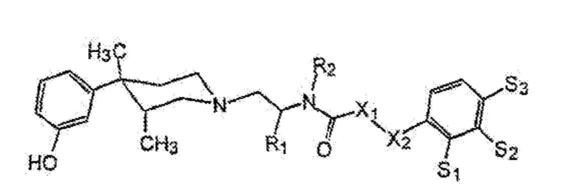 dans laquelle R₁, R₂, X₁, X₂, S₁, S₂, S₃ sont tels que définis dans le tableau suivant :
| Composé | R₁ | R₂ | X₁ | X₂ | S₁ | S₂ | S₃ |
|---|---|---|---|---|---|---|---|
| 8 | i-Pr | H | CH₂ | CH₂ | H | H | OH |
| 9 | i-Pr | H | CH₂ | CH₂ | H | H | OH |
| 10 | i-Pr | H | CH₂ | CH₂ | H | H | H |
| 11 | i-Pr | H | CH₂ | CH₂ | H | OH | H |
| 12 | i-Pr | H | CH₂ | CH₂ | OH | H | H |
| 13 | i-Pr | H | CH₂ | - | H | H | OH |
| 14 | i-Pr | H | CH | CH | H | H | OH |
| 15 | i-Pr | H | CH₂ | CH₂ | H | H | F |
| 16 | i-Pr | H | CH₂ | CH₂ | H | OH | OH |
| 17 | i-Pr | H | CH₂ | CH₂ | H | OCH₃ | OH |
| 18 | i-Pr | H | CH₂ | CH₂ | H | H | OCH₃ |
| 19 | H | H | CH₂ | CH₂ | H | H | OH |
et dans lequel, dans le composé 9, le carbone auquel le groupe i-Pr est fixé a la stéréochimie opposée de celle dans le composé 8 ; et dans le composé 14, la double liaison reliant X₁ et X₂ est une double liaison trans.

7. Composé de formule (I) selon la revendication 1, lequel composé est représenté par la formule suivante : 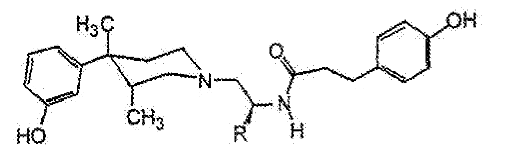 dans laquelle R est tel que défini dans le tableau suivant :
| Composé | R |
|---|---|
| 24 | i-Bu |
| 25 | sec-Bu |
| 26 | c-Hex |
| 27 | Benzyle |
